# EUROPEAN PATENT APPLICATION

(11) **EP 4 555 999 A1**
(43) Date of publication of application: **21.05.2025**
(21) Application number: 23839683.2
(22) Date of filing: 13.07.2023
(51) Int. Cl.: A61K 9/127, A61K 9/51, A61K 31/7088, A61K 47/10, A61K 47/22, A61K 47/28, A61K 47/34, A61P 37/04, C08K 5/05, C08K 5/1545, C08K 5/36, C08K 5/521, C08L 71/02

(54) **LIPID NANOPARTICLE AND METHOD FOR PRODUCING SAME, NUCLEIC ACID-ENCAPSULATED LIPID NANOPARTICLE AND METHOD FOR PRODUCING SAME, AND METHOD FOR INDUCING ACQUIRED IMMUNITY IN LIVING BODY BY USING NUCLEIC ACID-ENCAPSULATED LIPID NANOPARTICLE**

(30) Priority: 15.07.2022 JP 2022114395
(71) Applicant: NOF Corporation, Shibuya-ku Tokyo 150-6019 (JP); National University Corporation Chiba University, Chiba-shi, Chiba 263-8522 (JP)
(72) Inventor: NAKAI, Yuta, Kawasaki-shi, Kanagawa 210-0865 (JP); TANGE, Kota, Kawasaki-shi, Kanagawa 210-0865 (JP); AKITA, Hidetaka, Chiba-shi, Chiba 260-8675 (JP); TANAKA, Hiroki, Chiba-shi, Chiba 260-8675 (JP); SAKURAI, Yu, Chiba-shi, Chiba 260-8675 (JP); ANINDITA, Jessica, Chiba-shi, Chiba 260-8675 (JP); OYAMA, Ryotaro, Chiba-shi, Chiba 260-8675 (JP)
(74) Representative: Weickmann & Weickmann PartmbB
(86) International application number: PCT/JP2023/025891
(87) International publication number: WO 2024/014511

(57) **Abstract**

The present invention provides lipid nanoparticles containing
(A) an ionic lipid represented by the formula (1): 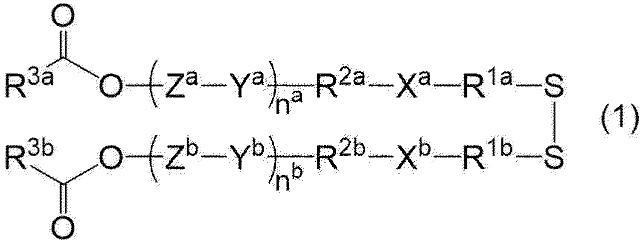 (the symbols in the formula (1) are as defined in the DESCRIPTION),
(B) vitamin E and/or a derivative thereof,
(C) phospholipid,
(D) cholesterol, and
(E) PEG lipid.

## Description

### [Technical Field]

The present invention relates to lipid nanoparticles particularly usable as nucleic acid vaccines and production methods thereof, nucleic acid-encapsulating lipid nanoparticles and production methods thereof, and methods for imparting acquired immunity to living body by using nucleic acid-encapsulating lipid nanoparticles.

### [Background Art]

For practicalization of nucleic acid therapy using oligonucleic acids such as siRNA, and gene therapy using mRNA, pDNA, and the like, an effective and safe nucleic acid delivery carrier is demanded. While virus vectors are nucleic acid delivery carriers with good expression efficiency, they have practical problems from the aspect of safety. Thus, the development of non-viral nucleic acid delivery carriers that can be used more safely is ongoing. Among them, lipid nanoparticles that are carriers using ionic lipids are non-viral nucleic acid delivery carriers most generally used at present.

Ionic lipids are largely constituted of amine moiety and lipid moiety. For example, the amine moiety of the ionic lipid, which is protonated under acidic conditions, interacts electrostatically with nucleic acids, which are polyanions, to form lipid nanoparticles, which promotes uptake into cells and delivers nucleic acids into cells.

A known ionic lipid that is generally widely used is, for example, 1,2-dioleoyloxy-3-dimethylaminopropane (DODAP). It is known that by combining such known ionic lipids with phospholipids, cholesterol, and PEG lipids, lipid nanoparticles can be formed and nucleic acids can be delivered into cells (Non Patent Literature 1).

For example, Patent Literature 1 shows an ionic lipid having a structure in which compounds consisting of one or two amine moieties and one lipid moiety are connected by a biodegradable disulfide bond. Patent Literature 1 shows that the ionic lipid can improve pharmacokinetics such as blood stability and tumor targeting. It also shows that by changing the structure around the amine moiety, the pKa of a lipid membrane structure can be adjusted to a value advantageous for endosomal escape in cells, and further that it has the effect of dissociating nucleic acids from lipid membrane structures by utilizing the cleavage of disulfide bonds within cells. In fact, since it shows higher nucleic acid delivery efficiency compared to a known ionic lipid DODAP, it is clear that this ionic lipid can achieve improvement of improve the intracellular dynamics such as improvement of the delivery efficiency of nucleic acids into the cytoplasm and the like.

For example, in Patent Literature 2, a lipid membrane structure is shown that has enhanced ability to fuse with endosomal membrane and has further improved efficiency of nucleic acid delivery into the cytoplasm, by using an ionic lipid having, in addition to a tertiary amine moiety and disulfide bond, an aromatic ring introduced near the lipid moiety.

Thus, ionic lipids with improved intracellular dynamics have been developed by increasing endosomal escape efficiency and membrane fusion ability. However, in order to increase the effect of lipid nanoparticles containing ionic lipids as vaccines, it is also an important factor to increase acquired immunity (cellular immunity and humoral immunity) in addition to nucleic acid delivery efficiency.

There are several examples in which lipid nanoparticles containing ionic lipids have been used as nucleic acid vaccines. For example, Non Patent Literature 2 shows an example in which 1,2-dioleoyloxy-3-trimethylammonium-propane (DOTAP) was used as the ionic lipid.

As described, nucleic acid vaccines using lipid nanoparticles have been developed, but the effect of enhancing acquired immunity is not sufficient and there is room for improvement.

### [Citation List]

### [Patent Literature]

[Patent Literature 1]
   US Patent No. 9708628
[Patent Literature 2]
   WO 2019/188867

### [Non Patent Literature]

[Non Patent Literature 1]
   Molecular Therapy, 25(7): 1467-1475 (2017)
[Non Patent Literature 2]
   Vaccine, 8(3): 433 (2020)
[Non Patent Literature 3]
   Hum Vaccin Immunother., 14(3): 550-564 (2018)
[Non Patent Literature 4]
   Immunity, 54(12): 2877-2892 (2021)

### [Summary of Invention]

### [Technical Problem]

As a method for enhancing the effectiveness of a vaccine, a method of adding an adjuvant to the constituent components of the vaccine is used. For example, O/W emulsions such as AS03 and MF59, aluminum salts (alum), and the like are used as adjuvants for influenza vaccines (see, for example, Non Patent Literature 3).

However, while these adjuvants can enhance the effect of vaccines, they also problematically increase side effects such as fever and pain at the administration site. Therefore, the selection of the adjuvant is also important.

Lipid nanoparticles are also suggested to have the possibility as adjuvants (e.g., Non Patent Literature 4). However, a sufficient effect of the lipid nanoparticles using ionic lipids, shown in Patent Literature 2, as a nucleic acid vaccine has not been shown. Thus, the effect of lipid nanoparticles as a vaccine constituent element is not fully satisfactory and needs to be investigated.

In view of the above-mentioned problems, the present invention aims to provide lipid nanoparticles that are particularly used for efficiently enhancing acquired immunity and production methods thereof, nucleic acid-encapsulating lipid nanoparticles, and production methods thereof, and methods for imparting acquired immunity to living body by using nucleic acid-encapsulating lipid nanoparticles.

### [Solution to Problem]

In view of the above-mentioned problems, the inventors have conducted extensive studies and found that lipid nanoparticles produced using an ionic lipid that has a pKa suitable for endosomal escape and that is specifically decomposed in a reducing environment within cells to enhance the efficiency of nucleic acid delivery into cells, and vitamin E or a vitamin E derivative that has adjuvant activity, can efficiently enhance acquired immunity, which resulted in the completion of the present invention. The present invention based on this finding is as follows.

[1] A lipid nanoparticle comprising
   (A) an ionic lipid represented by the formula (1): 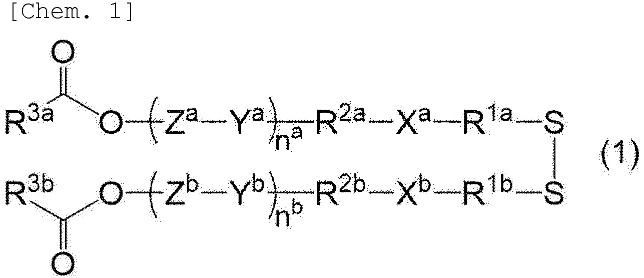 (in the formula (1),
      R^{1a} and R^{1b} are each independently an alkylene group having 1 to 6 carbon atoms,
      X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
      R^{2a} and R^{2b} are each independently an alkylene group or an oxydialkylene group each having not more than 8 carbon atoms,
      Y^{a} and Y^{b} are each independently an ester bond, an amide bond, a carbamate bond, an ether bond or a urea bond,
      Z^{a} and Z^{b} are each independently a divalent group derived from an aromatic compound having 3 to 16 carbon atoms and at least one aromatic ring, and optionally having a hetero atom,
      n^{a} and n^{b} are each independently 0 or 1, and
      R^{3a} and R^{3b} are each independently
         (a) a group such that R^{3a}-COO- and R^{3b}-COO- are each a residue derived from a reaction product of a sterol derivative having a hydroxyl group, and succinic anhydride or glutaric anhydride, or
         (b) an aliphatic hydrocarbon group having 1 to 40 carbon atoms, an alkyl group having 3 to 40 carbon atoms and a cyclopropane ring, or
         (c) a group represented by the formula (2):

            R⁹-O-CO-(CH₂)ₐ- (2)

            (in the formula (2),
            R⁹ is an aliphatic hydrocarbon group having 2 to 20 carbon atoms, and
            a is an integer of 2 to 10)),
   (B) vitamin E and/or a derivative thereof,
   (C) phospholipid,
   (D) cholesterol, and
   (E) PEG lipid.
[2] The lipid nanoparticle of [1], wherein the aforementioned vitamin E is selected from the group consisting of α-tocopherol, β-tocopherol, α-tocotrienol, and β-tocotrienol.
[3] The lipid nanoparticle of [1] or [2], wherein the aforementioned vitamin E derivative is selected from the group consisting of α-tocopherol succinate, α-tocopherol glutarate, β-tocopherol succinate, β-tocopherol glutarate, α-tocotrienol succinate, α-tocotrienol glutarate, β-tocotrienol succinate, and β-tocotrienol glutarate.
[4] The lipid nanoparticle of [1] or [2], wherein the aforementioned vitamin E derivative is a compound represented by the formula (3): [0020] 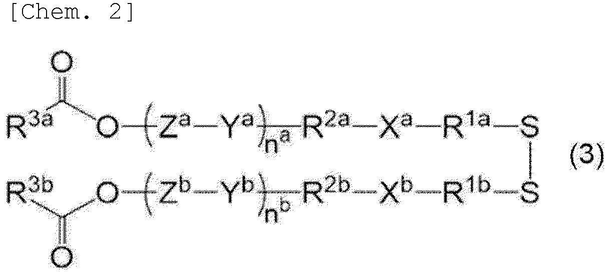 (in the formula (3),
   R^{1a} and R^{1b} are each independently an alkylene group having 1 to 6 carbon atoms,
   X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
   R^{2a} and R^{2b} are each independently an alkylene group or an oxydialkylene group each having not more than 8 carbon atoms,
   Y^{a} and Y^{b} are each independently an ester bond, an amide bond, a carbamate bond, an ether bond or a urea bond,
   Z^{a} and Z^{b} are each independently a divalent group derived from an aromatic compound having 3 to 16 carbon atoms and at least one aromatic ring, and optionally having a hetero atom,
   n^{a} and n^{b} are each independently 0 or 1,
   R^{3a} and R^{3b} are each independently a group such that R^{3a}-COO- and R^{3b}-COO- are each a residue derived from a reaction product of a hydroxyl group of vitamin E, and succinic anhydride or glutaric anhydride).
[5] The lipid nanoparticle of [1] or [2], wherein the aforementioned vitamin E derivative is a compound represented by the formula (4): [0022] 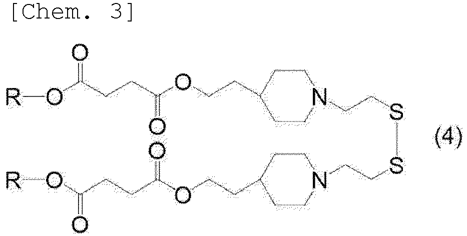 (in the formula (4),
   R is a group such that RCOO(CH₂)₂COO- is a residue of a reaction of a hydroxyl group of α-tocopherol, β-tocopherol, α-tocotrienol, or β-tocotrienol and succinic anhydride) or the formula (5): 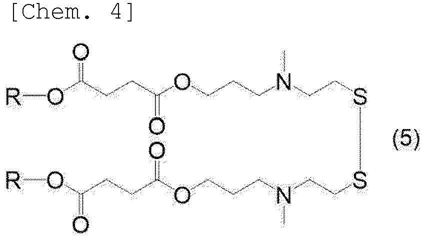 (in the formula (5),
   R is a group such that RCOO(CH₂)₂COO- is a residue of a reaction of a hydroxyl group of α-tocopherol, β-tocopherol, α-tocotrienol, or β-tocotrienol and succinic anhydride).
[6] The lipid nanoparticle of any of [1] to [5], wherein the aforementioned ionic lipid is a compound represented by the following formula: 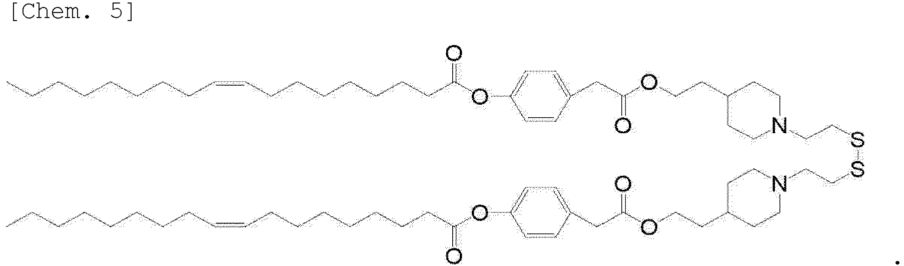
[7] The lipid nanoparticle of [5], comprising 20 to 55 mol% of the aforementioned ionic lipid, 4.5 to 35 mol% of the aforementioned vitamin E and/or a derivative thereof, 5 to 35 mol% of the aforementioned phospholipid, 10 to 50 mol% of the aforementioned cholesterol, and 0.5 to 4 mol% of the aforementioned PEG lipid, with respect to the total of the aforementioned ionic lipid, the aforementioned vitamin E and/or a derivative thereof, the aforementioned phospholipid, and the aforementioned cholesterol.
[8] The lipid nanoparticle of [6], comprising 20 to 55 mol% of the aforementioned ionic lipid, 4.5 to 35 mol% of the aforementioned vitamin E and/or a derivative thereof, 5 to 35 mol% of the aforementioned phospholipid, 10 to 50 mol% of the aforementioned cholesterol, and 0.5 to 4 mol% of the aforementioned PEG lipid, with respect to the total of the aforementioned ionic lipid, the aforementioned vitamin E and/or a derivative thereof, the aforementioned phospholipid, and the aforementioned cholesterol.
[9] The lipid nanoparticle of [8], wherein the aforementioned phospholipid is 1,2-diacyl-sn-glycero-3-phosphocholine or 1,2-diacyl-sn-glycero-phosphoethanolamine.
[10] The lipid nanoparticle of [8], wherein the aforementioned phospholipid is selected from the group consisting of 1,2-dioleoyl-sn-glycero-3-phosphocholine (DOPC), 1,2-distearoyl-sn-glycero-3-phosphocholine (DSPC), and 1,2-dioleoyl-sn-glycero-3-phosphoethanolamine (DOPE).
[11] The lipid nanoparticle of [8], wherein the aforementioned PEG lipid is a dimyristoylglycerol PEG represented by the formula (6):

   CH₂(OR⁶)-CH(OR⁷)-CH₂(OR⁸) (6)

   (in the formula (6),
   any two of R⁶, R⁷ and R⁸ are myristoyl groups, and the remaining one is an alkyl group having 1 to 6 carbon atoms which is linked via a polyethylene glycol chain having a number average molecular weight of 1,000 to 3,000).
[12] The lipid nanoparticle of [10], wherein the aforementioned PEG lipid is a dimyristoylglycerol PEG represented by the formula (6):

   CH₂(OR⁶)-CH(OR⁷)-CH₂(OR⁸) (6)

   (in the formula (6),
   any two of R⁶, R⁷ and R⁸ are myristoyl groups, and the remaining one is an alkyl group having 1 to 6 carbon atoms which is linked via a polyethylene glycol chain having a number average molecular weight of 1,000 to 3,000).
[13] A nucleic acid-encapsulating lipid nanoparticle comprising the lipid nanoparticle of any of [1] to [12] and a nucleic acid encapsulated therein.
[14] A method for production the nucleic acid-encapsulating lipid nanoparticle of [13], comprising a vitamin addition step for adding the aforementioned vitamin E and/or a derivative thereof to the aforementioned ionic lipid, the aforementioned phospholipid, the aforementioned cholesterol, and the aforementioned PEG lipid.
[15] The production method of [14], wherein the aforementioned vitamin addition step further comprises, after addition of the aforementioned vitamin E and/or a derivative thereof to an alcohol solution comprising the aforementioned ionic lipid, the aforementioned phospholipid, the aforementioned cholesterol, and the aforementioned PEG lipid, a step of mixing with a buffer comprising the nucleic acid.
[16] The production method of [14], wherein the aforementioned vitamin addition step comprises, after mixing an alcohol solution comprising the aforementioned ionic lipid, the aforementioned phospholipid, the aforementioned cholesterol, and the aforementioned PEG lipid with a buffer comprising the nucleic acid, adding the aforementioned vitamin E and/or a derivative thereof.
[17] A method for imparting a living body with an acquired immunity to an antigen protein, comprising administering the lipid nanoparticle of any of [1] to [12] encapsulating a nucleic acid that expresses the antigen to the living body.

### [Advantageous Effects of Invention]

The lipid nanoparticles of the present invention that are used for a nucleic acid vaccine that can particularly efficiently enhance acquired immunity have a pKa suitable for endosomal escape and can efficiently enhance acquired immunity as compared with lipid nanoparticles of the prior art, by combining ionic lipid that is specifically decomposed in a reducing environment within cells to enhance the efficiency of nucleic acid delivery into cells, with at least one of vitamin E having an adjuvant ability and a vitamin E derivative having the vitamin E skeleton.

### [Brief Description of Drawings]

[Fig. 1]

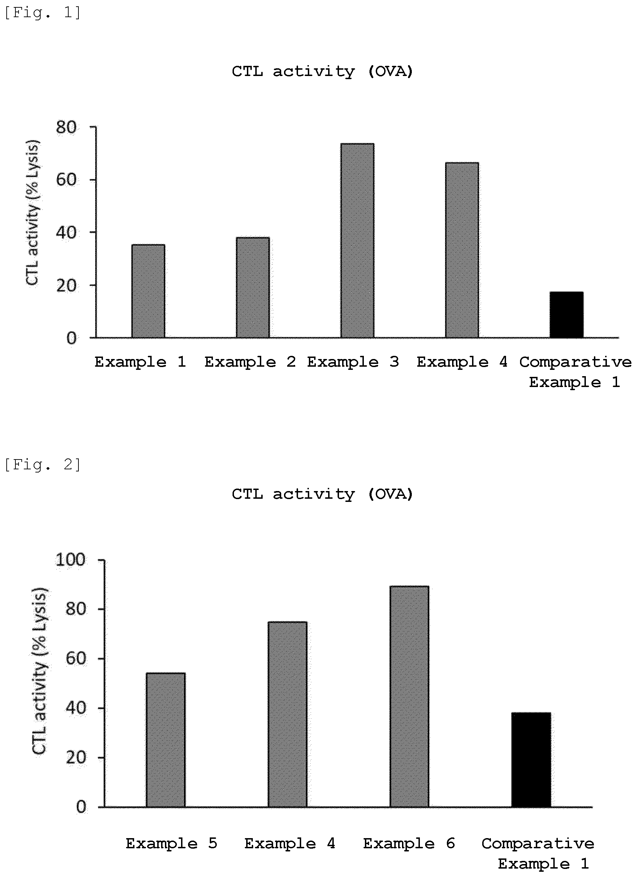

   Fig. 1 shows the effect of adding α-tocopherol (VE) to lipid nanoparticles on CTL activity in mice administered with the lipid nanoparticles. Regardless of the timing of VE addition, CTL activity increased compared to the case without VE addition.
[Fig. 2]
   Fig. 2 shows the effect of addition concentration of VE on CTL activity in mice administered with lipid nanoparticles.
[Fig. 3]

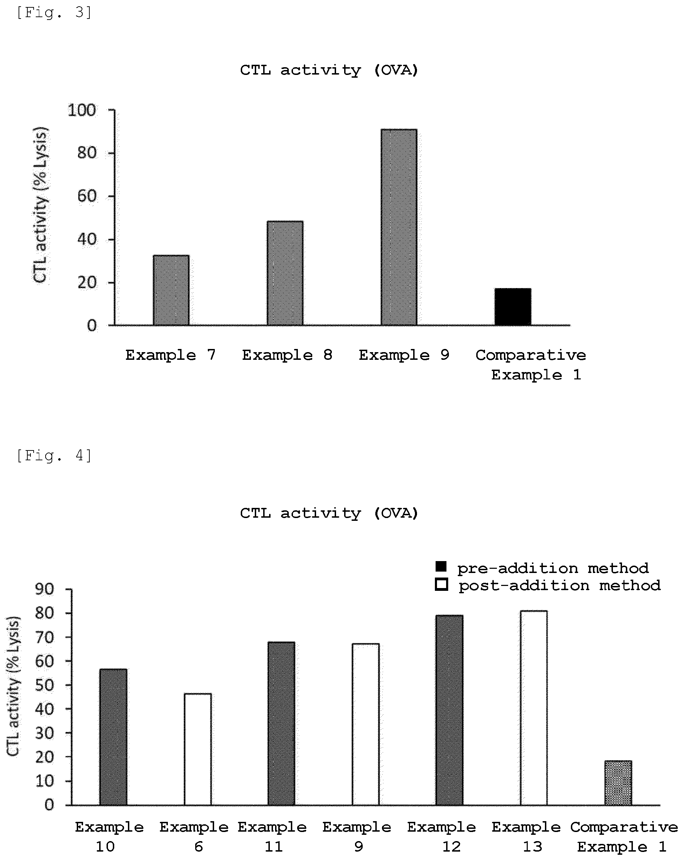

   Fig. 3 shows the effect of addition concentration of α-tocopherol succinate (TS) on CTL activity in mice administered with lipid nanoparticles.
[Fig. 4]
   Fig. 4 shows the results of examining the effect of the method of adding vitamins/vitamin derivatives (VE, TS, SS-EC) on CTL activity in mice administered with lipid nanoparticles. Regardless of the method of addition, CTL activity increased compared to the case without addition of vitamin/vitamin derivative.
[Fig. 5]

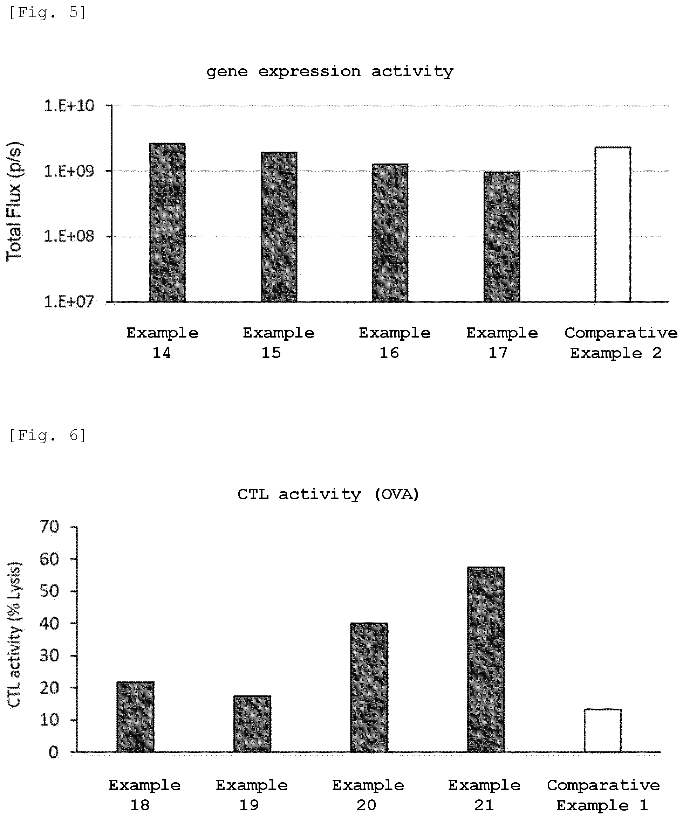

   Fig. 5 shows the effect of addition concentration of SS-EC on nucleic acid delivery efficiency (transgene expression activity) in mice administered with lipid nanoparticles. At all addition concentrations, the nucleic acid delivery efficiency equivalent to that without addition was obtained.
[Fig. 6]
   Fig. 6 shows the effect of addition concentration of SS-EC on CTL activity in mice administered with lipid nanoparticles. At all addition concentrations, the CTL activity increased as compared with the case of no addition.
[Fig. 7]

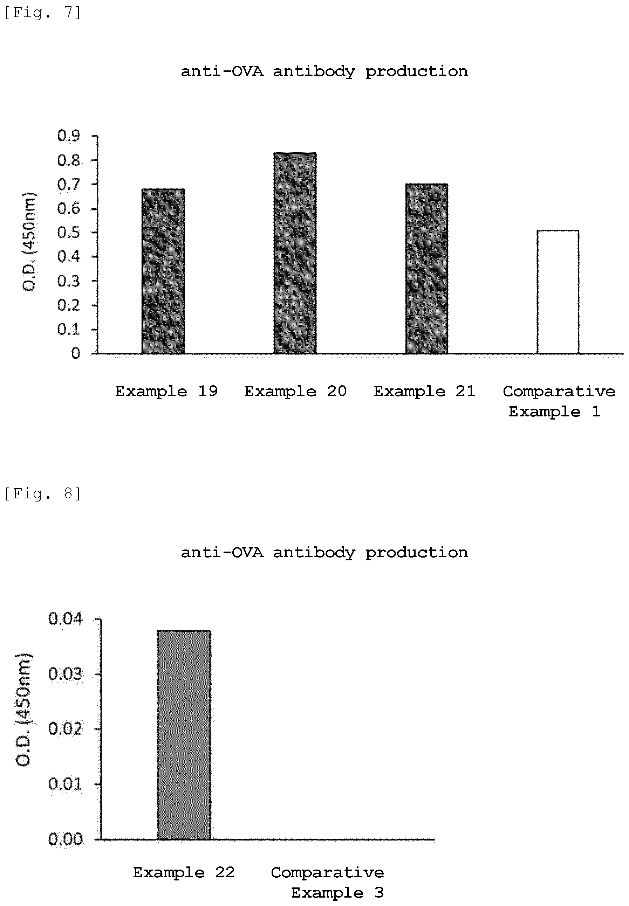

   Fig. 7 shows the effect of addition concentration of SS-EC on anti-OVA antibody production in mice administered with lipid nanoparticles. At all addition concentrations, the antibody production quantity increased as compared with the case of no addition.
[Fig. 8]
   Fig. 8 shows the effect of addition of SS-EC to lipid nanoparticles on the anti-OVA antibody production quantity in mice administered with the lipid nanoparticles. In the case of SS-EC no addition, antibody was not produced but SS-EC addition induced antibody production.
[Fig. 9]

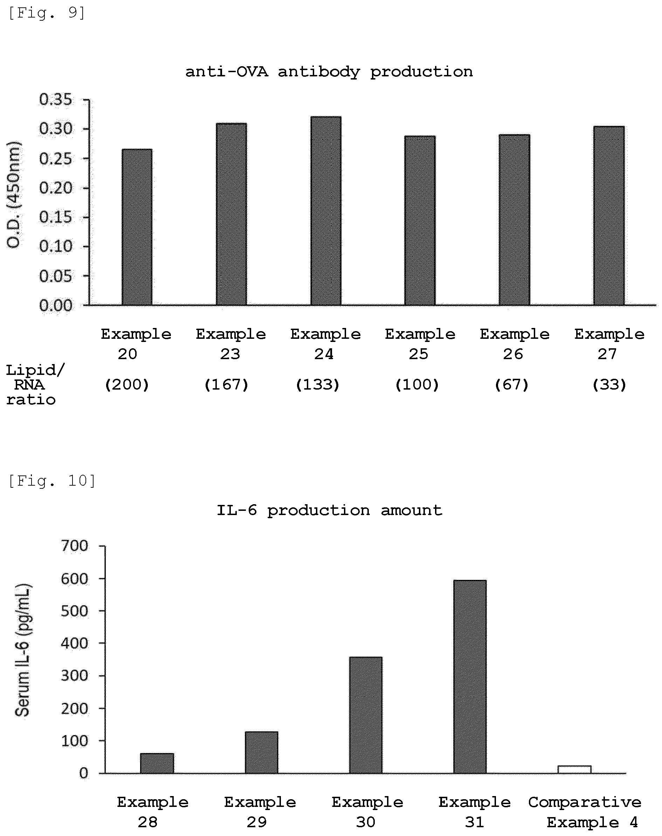

   Fig. 9 shows the effect of the ratio of RNA for the total lipid amount (L/R ratio) on anti-OVA antibody production in mice administered with lipid nanoparticles. At all L/R ratios, equivalent antibody production quantity was observed.
[Fig. 10]
   Fig. 10 shows the effect of addition concentration of SS-EC on IL-6 production in mice administered with lipid nanoparticles. At all addition concentrations, the IL-6 production quantity increased as compared with the case of no addition.

### [Description of Embodiments]

While the embodiments of the present invention are explained in the following, the present invention is not limited thereto. The present invention relates to a lipid nanoparticle containing
(A) an ionic lipid represented by the formula (1) (i.e., ionic lipid having a tertiary amino group, a lipid moiety, and a disulfide bond as a biodegradable group),
(B) vitamin E and/or a derivative thereof,
(C) phospholipid,
(D) cholesterol, and
(E) PEG lipid,
which is particularly useful as a constituent element of vaccine (hereinafter sometimes to be referred to as "the lipid nanoparticles of the present invention") and a production method thereof, a nucleic acid-encapsulating lipid nanoparticle and a production method thereof, and a method for imparting a living body with an acquired immunity to an antigen protein by using a nanoparticle encapsulating a nucleic acid that expresses the antigen.

### Lipid nanoparticles

In the present specification, the "lipid nanoparticles" (Lipid Nano Particle, sometimes to be abbreviated as "LNP" in the present specification) means a particle having a membrane structure wherein the hydrophilic groups of amphiphilic lipids are arranged in the interface, facing the aqueous phase side, and having a particle size of less than 1 µm. The "amphiphilic lipid" means a lipid having both a hydrophilic group and a hydrophobic group.

The particle size of the lipid nanoparticle of the present invention is preferably 10 nm to 500 nm, more preferably 30 nm to 300 nm. The particle size can be measured by using a particle size distribution measuring device such as Zetasizer Nano (Malvern) or the like. The particle size of the lipid nanoparticles can be appropriately adjusted by the method for producing the lipid nanoparticles. In the present specification, the "particle size" means an average particle size (zeta average) measured by a dynamic light scattering method.

Examples of the amphiphilic lipid include ionic lipid, phospholipid, PEG lipid, and the like. In the present specification, "PEG" means polyethylene glycol, "PEG lipid" means a lipid modified with PEG, and "Y modified with X " (e.g., X: PEG, Y: lipid) means Y bound by X. In other words, "PEG lipid" means a lipid bound by PEG.

### Ionic lipid

The ionic lipid used in the present invention is an ionic lipid represented by the following formula (1) (sometimes to be abbreviated as "ionic lipid (1)" in the present specification). Only one kind of ionic lipid (1) may be used, or two or more kinds thereof may be used in combination. 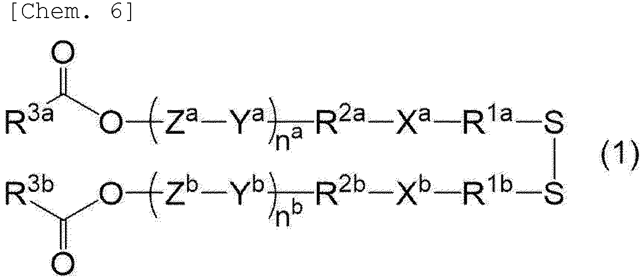 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1, and
R^{3a} and R^{3b} are each independently
   (a) a group such that R^{3a}-COO- and R^{3b}-COO- are each a residue derived from a reaction product of a sterol derivative having a hydroxyl group, and succinic anhydride or glutaric anhydride, or
   (b) an aliphatic hydrocarbon group having 1 to 40 carbon atoms, an alkyl group having 3 to 40 carbon atoms and a cyclopropane ring, or
   (c) a group represented by the formula (2):

      R⁹-O-CO-(CH₂)ₐ- (2)

      (in the formula (2),
      R⁹ is an aliphatic hydrocarbon group having 2 to 20 carbon atoms, and
      a is an integer of 2 to 10).

R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group, and the like. Preferably, R^{1a} and R^{1b} are each independently a methylene group, an ethylene group, a trimethylene group, an isopropylene group, or a tetramethylene group, most preferably an ethylene group.

R^{1a} may be the same as or different from R^{1b}, and R^{1a} is preferably the same group as R^{1b}.

X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

A preferred specific structure of the acyclic alkyl tertiary amino group having 1 to 6 carbon atoms and one tertiary amino group is represented by X¹.

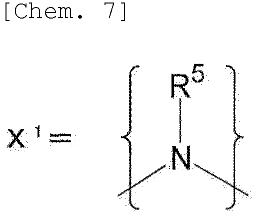

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridylene group, an azetidylene group, a pyrrolidylene group, a piperidylene group, an imidazolidylene group, or a piperazylene group, preferably a pyrrolidylene group, a piperidylene group, or a piperazylene group, most preferably a piperidylene group.

A preferred specific structure of the cyclic alkylene tertiary amino group having 2 to 5 carbon atoms and one tertiary amino group is represented by X².

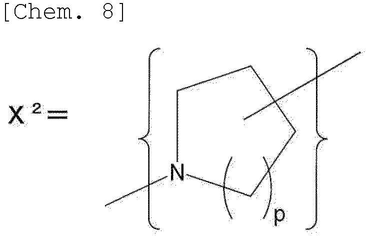

The p in X² is 1 or 2. When p is 1, X² is a pyrrolidylene group, and when p is 2, X² is a piperidylene group. Preferably, p is 2.

A preferred specific structure of the cyclic alkylene tertiary amino group having 2 to 5 carbon atoms and two tertiary amino groups is represented by X³.

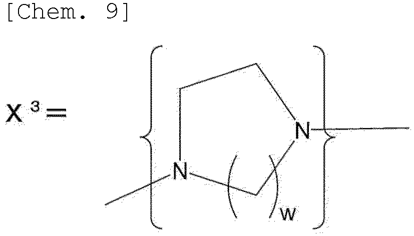

The w in X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group, and the like. It is preferably a methylene group, an ethylene group, a trimethylene group, or a tetramethylene group, most preferably an ethylene group.

In the present specification, the "oxydialkylene group having not more than 8 carbon atoms" means alkylene groups via an ether bond (alkylene-O-alkylene, in other words, "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. It is preferably an oxydimethylene group, an oxydiethylene group, or an oxydi(tetramethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, and anthracene ring can be mentioned for aromatic hydrocarbon ring, and imidazole ring, pyrazole ring, oxazole ring, isoxazole ring, thiazole ring, isothiazole ring, triazine ring, pyrrole ring, furan thiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxazine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 2 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. 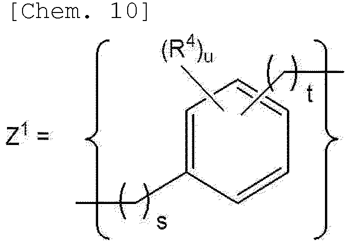 wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of the ionic lipid. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently
(a) a group such that R^{3a}-COO- and R^{3b}-COO- are each a residue derived from a reaction of a sterol derivative having a hydroxyl group, and succinic anhydride or glutaric anhydride,
(b) an aliphatic hydrocarbon group having 1 to 40 carbon atoms, an alkyl group having 3 to 40 carbon atoms and a cyclopropane ring, or
(c) a group represented by the formula (2):

   R⁹-O-CO-(CH₂)ₐ- (2)

   (in the formula (2),
   R⁹ is an aliphatic hydrocarbon group having 2 to 20 carbon atoms, and
   a is an integer of 2 to 10),
   preferably each independently an aliphatic hydrocarbon group having 12 to 22 carbon atoms.

The residue derived from a reaction product of a sterol derivative having a hydroxyl group, and succinic anhydride or glutaric anhydride is a group having a structure in which the hydroxyl group of the sterol derivative having the hydroxyl group is replaced with *-O-CO-CH₂-CH₂- or *-O-CO-CH₂-CH₂-CH₂-. * shows the bonding position with the sterol derivative.

Examples of the sterol derivative having a hydroxyl group include cholesterol, cholestanol, stigmasterol, β-sitosterol, lanosterol, ergosterol, and the like. It is preferably cholesterol or cholestanol.

The aliphatic hydrocarbon group having 1 to 40 carbon atoms may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 12 to 22, more preferably 13 to 19, most preferably 13 to 17. While the aliphatic hydrocarbon group includes an alkyl group, an alkenyl group, an alkynyl group, and the like, it is preferably an alkyl group or an alkenyl group. Specific examples of the aliphatic hydrocarbon group having 1 to 40 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, henicosyl group, docosyl group, tricosyl group, tetracosyl group, pentacosyl group, hexacosyl group, heptacosyl group, octacosyl group, nonacosyl group, triacontyl group, tetracontyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, decadienyl group, tridecadienyl group, tetradecadienyl group, pentadecadienyl group, hexadecadienyl group, heptadecadienyl group, octadecadienyl group, nonadecadienyl group, icosadienyl group, henicosadienyl group, docosadienyl group, octadecatrienyl group, icosatrienyl group, icosatetraenyl group, icosapentaenyl group, docosahexaenyl group, isostearyl group, 1-hexylheptyl group, 1-hexylnonyl group, 1-octylnonyl group, 1-octylundecyl group, 1-decylundecyl group, and the like. The aliphatic hydrocarbon group having 1 to 40 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In one embodiment of the present invention, the aliphatic hydrocarbon group having 1 to 40 (preferably 12 to 22) carbon atoms for R^{3a} or R^{3b} is derived from fatty acid. In this case, the carbonyl carbon derived from fatty acid is contained in - CO-O- in the formula (1). A specific example of the aliphatic hydrocarbon group is a heptadecadienyl group when linoleic acid is used as the fatty acid, or a heptadecenyl group when oleic acid is used as the fatty acid.

The alkyl group having 3 to 40 carbon atoms and a cyclopropane ring for R^{3a} or R^{3b} mean an alkyl group having 3 to 40 carbon atoms having at least one cyclopropane ring in the alkyl chain. The carbon number 3 to 40 in the alkyl group does not include the number of carbon atoms in the cyclopropane ring. The number of cyclopropane rings in the alkyl group is preferably one. The alkyl group having 3 to 40 carbon atoms and a cyclopropane ring for R^{3a} or R^{3b} is preferably a group represented by the formula (6): 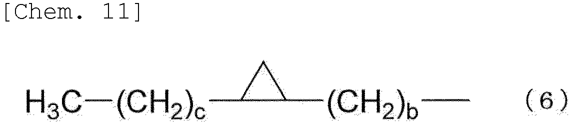 (in the formula (6), b and c are each independently an integer and the total of b and c is 2 to 39). Preferably, b is an integer of 1 to 20 and c is an integer of 1 to 19. b is more preferably an integer of 2 to 18, further preferably an integer of 3 to 17, further more preferably an integer of 4 to 12. c is more preferably an integer of 3 to 15, further preferably an integer of 3 to 11, further more preferably an integer of 3 to 9. Examples of the group represented by the formula (6) include 7-(2-octylcyclopropyl)heptyl and the like.

The aliphatic hydrocarbon group having 2 to 20 carbon atoms, which is represented by R⁹ in the formula (2) may be linear or branched. The aliphatic hydrocarbon group may be saturated or unsaturated. In the case of an unsaturated aliphatic hydrocarbon group, the aliphatic hydrocarbon group generally contains 1 to 6, preferably 1 to 3, more preferably 1 to 2 unsaturated bonds. While the unsaturated bond includes a carbon-carbon double bond and a carbon-carbon triple bond, it is preferably a carbon-carbon double bond. The aliphatic hydrocarbon group has a carbon number of preferably 8 to 20, more preferably 9 to 19, further preferably 13 to 19, most preferably 13 to 17. While the aliphatic hydrocarbon group includes an alkyl group, an alkenyl group, an alkynyl group and the like, it is preferably an alkyl group or an alkenyl group, more preferably an alkyl group. Specific examples of the aliphatic hydrocarbon group having 2 to 20 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, hexyl group, heptyl group, octyl group, nonyl group, decyl group, undecyl group, dodecyl group, tridecyl group, tetradecyl group, pentadecyl group, hexadecyl group, heptadecyl group, octadecyl group, nonadecyl group, icosyl group, dodecenyl group, tridecenyl group, tetradecenyl group, pentadecenyl group, hexadecenyl group, heptadecenyl group, octadecenyl group, nonadecenyl group, icosenyl group, henicosenyl group, docosenyl group, dodecadienyl group, tridecadienyl group, tetradecadienyl group, pentadecadienyl group, hexadecadienyl group, heptadecadienyl group, octadecadienyl group, nonadecadienyl group, icosadienyl group, icosatrienyl group, icosatetraenyl group, icosapentaenyl group, isostearyl group, 1-hexylheptyl group, 1-ethylnonyl group, 1-butylnonyl group, 1-hexylnonyl group, 1-octylnonyl group, 1-octylundecyl group, 3-octylundecyl group, and the like. The aliphatic hydrocarbon group having 2 to 20 carbon atoms is preferably a tridecyl group, a pentadecyl group, a heptadecyl group, a nonadecyl group, a heptadecenyl group, a heptadecadienyl group, or a 1-hexylnonyl group, particularly preferably a tridecyl group, a heptadecyl group, a heptadecenyl group, or a heptadecadienyl group.

In the formula (2), a is preferably an integer of 3 to 9, more preferably an integer of 3 to 7, further preferably an integer of 5 to 7, most preferably 5 or 7.

R^{3a} may be the same as or different from R^{3b}, and R^{3a} is preferably the same group as R^{3b}.

In one embodiment of the present invention, R^{1a} is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of ionic lipid (1) include the following ionic lipids.

### [Ionic lipid (1-1)]

Ionic lipid (1) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently an aliphatic hydrocarbon group having 12 to 22 carbon atoms (e.g., heptadecenyl group, heptadecadienyl group, 1-hexylnonyl group).

### [Ionic lipid (1-2)]

Ionic lipid (1) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidylene group);
R^{2a} and R^{2b} are each independently an alkylene group having not more than 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently an aliphatic hydrocarbon group having 13 to 19 carbon atoms (e.g., heptadecenyl group, heptadecadienyl group, 1-hexylnonyl group).

### [Ionic lipid (1-3)]

Ionic lipid (1) wherein
R^{1a} and R^{1b} are each independently an alkylene group having 1 to 2 carbon atoms (i.e., methylene group or ethylene group);
X^{a} and X^{b} are each independently X¹: 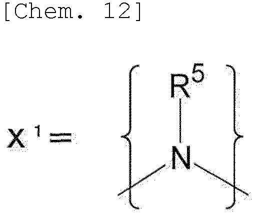 wherein R⁵ is an alkyl group having 1 to 3 carbon atoms (e.g., methyl group), or X²: 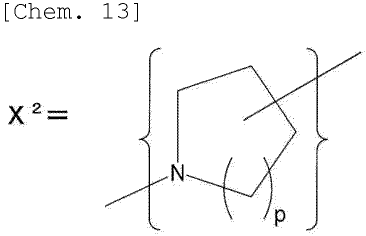 wherein p is 1 or 2;
R^{2a} and R^{2b} are each independently alkylene group having not more than 4 carbon atoms (e.g., methylene group, ethylene group, trimethylene group);
Y^{a} and Y^{b} are each independently an ester bond or an amide bond;
Z^{a} and Z^{b} are each independently Z¹: 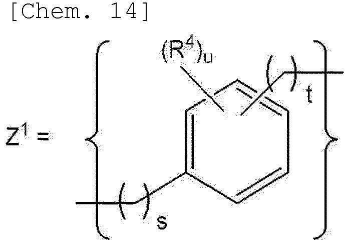 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently an aliphatic hydrocarbon group having 13 to 17 carbon atoms (e.g., heptadecenyl group, heptadecadienyl group, 1-hexylnonyl group).

Specific examples of ionic lipid (1) include the following O-Ph-P3C1, O-Ph-P4C1, O-Ph-P4C2, O-Bn-P4C2, L-Ph-P4C2, HD-Ph-P4C2, O-Ph-amide-P4C2, O-Ph-C3M, and O-P4C2.

Specific examples of ionic lipid (1) include Lipid 1 to Lipid 20 described in WO 2021/195529 A2, particularly, the following Lipid 1, Lipid 5, and Lipid 8.

**[Table 1-1]**

| name of ionic lipid | structure |
|---|---|
| O-Ph-P3C1 | 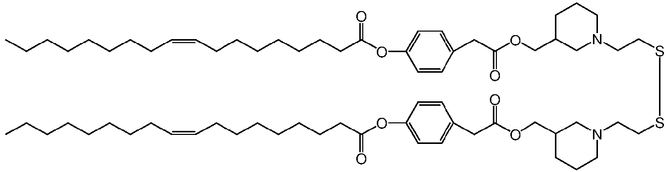 |
| O-Ph-P4C1 | 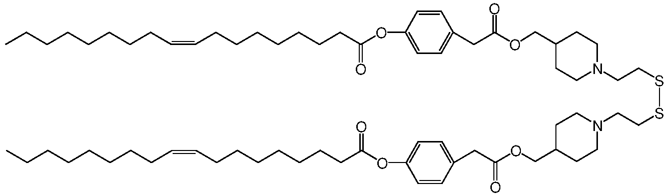 |
| O-Ph-P4C2 (or SS-OP) | 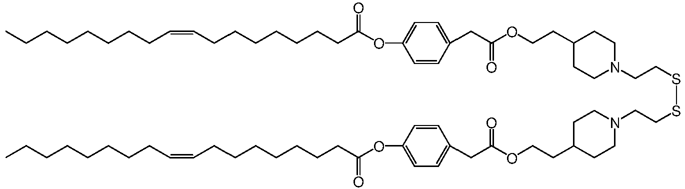 |
| O-Bn-P4C2 | 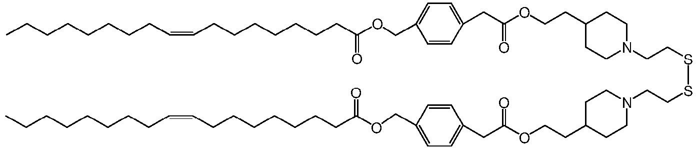 |

**[Table 1-2]**

| name of ionic lipid | structure |
|---|---|
| L-Ph-P4C2 | 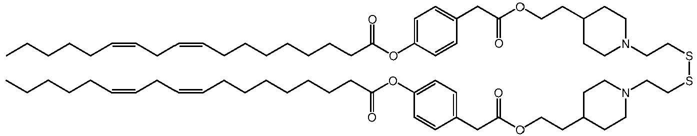 |
| HD-Ph-P4C2 | 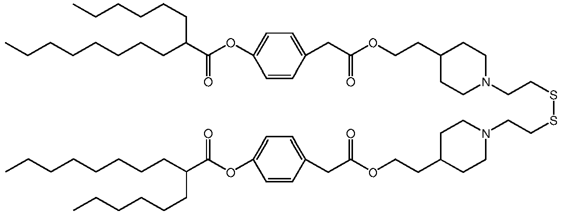 |
| O-Ph-amide-P4C2 | 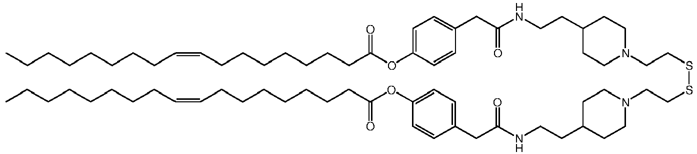 |
| O-Ph-C3M | 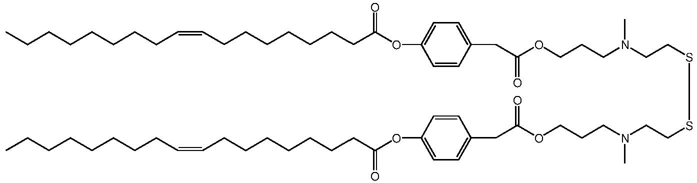 |
| O-P4C2 (or SS-OC) | 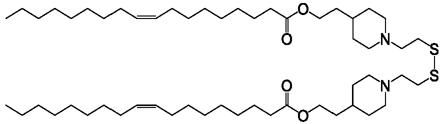 |

**[Table 1-3]**

| name of ionic lipid | structure |
|---|---|
| Lipid 1 | 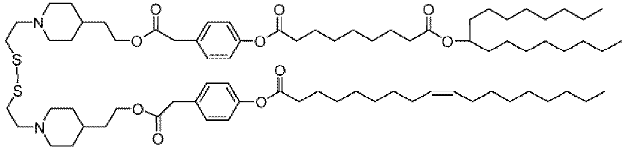 |
| Lipid 5 | 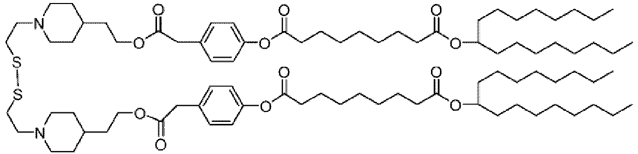 |
| Lipid 8 | 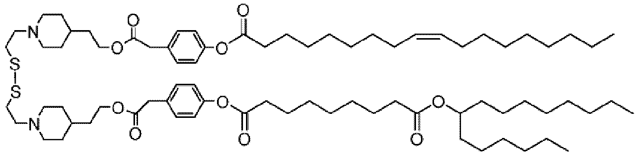 |

Among the specific examples of ionic lipid (1), SS-OP is preferred. That is, ionic lipid (1) is preferably an ionic lipid represented by the following formula.

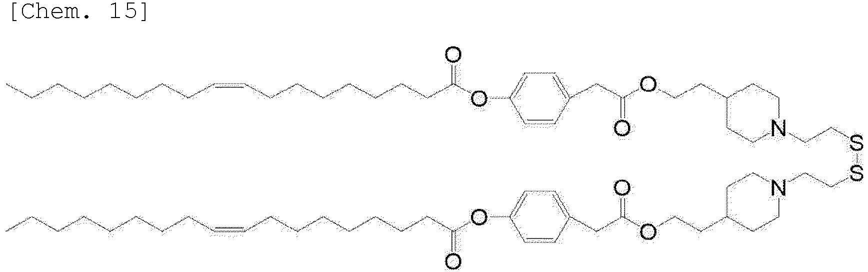

The amount of ionic lipid (1) in the lipid nanoparticles of the present invention when using vitamin E and/or a derivative thereof is preferably 20 to 55 mol%, more preferably 22.5 to 50 mol%, with respect to the total of ionic lipid (1), vitamin E and/or a derivative thereof, phospholipid, and cholesterol, from the aspects of efficiency of nucleic acid encapsulation, efficiency of intracellular release of nucleic acid, and stability of the lipid nanoparticles.

The ionic lipid (1) can be produced by a known method (e.g., the method described in WO 2019/188867 A1, US 9708628 B2, WO 2021/195529 A2).

### Vitamin E and vitamin E derivative

The lipid nanoparticles of the present invention contain at least one of vitamin E and vitamin E derivative as a constituent component of lipid. Only one kind of these may be used, or two or more kinds thereof may be used in combination.

### Vitamin E

Vitamin E is a collective term for 4 types of tocopherols and 4 types of tocotrienols, and specifically includes α-tocopherol, β-tocopherol, γ-tocopherol, δ-tocopherol, α-tocotrienol, β-tocotrienol, γ-tocotrienol, and δ-tocotrienol.

As the vitamin E to be used in the present invention, any one of the above-mentioned eight types may be used, or a combination of a plurality thereof may be used in combination. It is preferably α-tocopherol, β-tocopherol, α-tocotrienol, or β-tocotrienol, most preferably α-tocopherol.

### Vitamin E derivative

The compound is not limited as long as it has a vitamin E skeleton, and may be, for example, a compound in which the hydroxyl group of vitamin E is modified, a compound in which the isoprenoid side chain of vitamin E is modified (for example, a compound in which the terminal is carboxylated or a compound in which a hydroxyl group is introduced into the terminal), or a compound in which the chroman ring of vitamin E is modified. Among them, a compound in which the hydroxyl group of vitamin E is modified is preferred. Examples of the compound in which the hydroxyl group of vitamin E is modified include compounds in which vitamin E is reacted with an acid such as carboxylic acid, phosphoric acid or a derivative thereof, sulfonic acid, or the like to esterify the hydroxyl group. As the carboxylic acid, aliphatic carboxylic acids such as acetic acid, propionic acid, hexanoic acid, myristic acid, stearic acid, oleic acid, and the like, dibasic acids such as oxalic acid, malonic acid, succinic acid, glutar acid, adipic acid, fumaric acid, maleic acid, and the like, various amino acids, and the like can be mentioned. As the phosphoric acid derivative, phosphoric acid monoalkyl ester, phosphoric acid dialkyl ester, and the like can be mentioned. As the sulfonic acid, alkylsulfonic acid and the like can be mentioned.

Specific examples of the compound in which the hydroxyl group of vitamin E is esterified include α-tocopherol fatty acid esters such as α-tocopheryl acetate and the like; α-tocopherol phosphate; α-tocopherol dibasic acid esters (mono or diester) such as α-tocopherol malonate (mono or diester), α-tocopherol succinate (mono or diester), α-tocopherol glutarate (mono or diester), and the like; and other examples include compounds in which vitamin E is reacted with another compound via a dibasic acid, such as the compound represented by the below-mentioned formula (3). In the present invention, a compound represented by the below-mentioned formula (3) is preferred.

The aforementioned vitamin E derivative may be in a salt form, and salts with organic bases, salts with inorganic bases, salts with organic acids, and salts with inorganic acids can be mentioned.

Only one kind of vitamin E derivative may be used, or two or more kinds thereof may be used in combination.

In one preferred embodiment, the vitamin E derivative is a compound represented by the following formula (3) (sometimes to be abbreviated as "compound (3)" in the present specification). 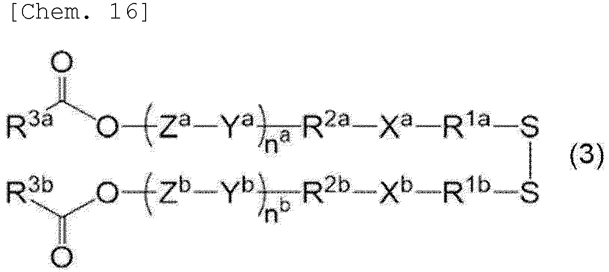 (in the formula (3),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1, and
R^{3a} and R^{3b} are each independently a group such that R^{3a}-COO- and R^{3b}-COO- are each a residue derived from a reaction product of a hydroxyl group of vitamin E, and succinic anhydride or glutaric anhydride).

R^{1a} and R^{1b} are each independently an alkylene group having 1 to 6 carbon atoms, and may be linear or branched, preferably linear. The carbon number of the alkylene group is preferably 1 to 4, more preferably 1 to 2. Specific examples of the alkylene group having 1 to 6 carbon atoms include methylene group, ethylene group, trimethylene group, isopropylene group, tetramethylene group, isobutylene group, pentamethylene group, neopentylene group and the like. Preferably, R^{1a} and R^{1b} are each independently a methylene group, an ethylene group, a trimethylene group, an isopropylene group or a tetramethylene group, most preferably an ethylene group.

R^{1a} may be the same as or different from R^{1b}, and R^{1a} is preferably the same group as R^{1b}.

X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups, preferably each independently a cyclic alkylene tertiary amino group having 2 to 5 carbon atoms and 1 to 2 tertiary amino groups.

The alkyl group having 1 to 6 carbon atoms in the acyclic alkyl tertiary amino group having 1 to 6 carbon atoms and one tertiary amino group may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

A preferred specific structure of the acyclic alkyl tertiary amino group having 1 to 6 carbon atoms and one tertiary amino group is represented by X¹.

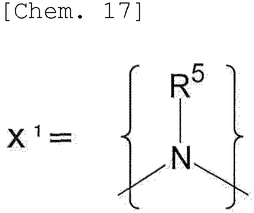

R⁵ in X¹ is an alkyl group having 1 to 6 carbon atoms, which may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group, an ethyl group, a propyl group, or an isopropyl group, most preferably a methyl group.

The carbon number of the cyclic alkylene tertiary amino group having 2 to 5 carbon atoms and 1 to 2 tertiary amino groups is preferably 4 to 5. The cyclic alkylene tertiary amino group having 2 to 5 carbon atoms and 1 to 2 tertiary amino groups is specifically an aziridylene group, an azetidylene group, a pyrrolidylene group, a piperidylene group, an imidazolidylene group, or a piperazylene group, preferably a pyrrolidylene group, a piperidylene group, or a piperazylene group, most preferably a piperidylene group.

A preferred specific structure of the cyclic alkylene tertiary amino group having 2 to 5 carbon atoms and one tertiary amino group is represented by X².

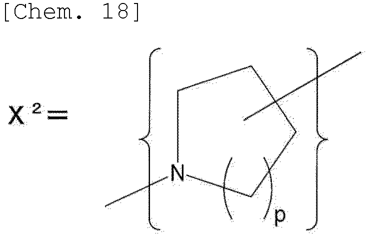

The p in X² is 1 or 2. When p is 1, X² is a pyrrolidylene group, and when p is 2, X² is a piperidylene group. Preferably, p is 2.

A preferred specific structure of the cyclic alkylene tertiary amino group having 2 to 5 carbon atoms and two tertiary amino groups is represented by X³.

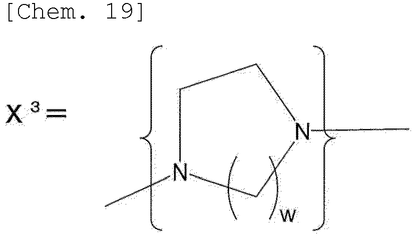

The w in X³ is 1 or 2. When w is 1, X³ is an imidazolidylene group, and when w is 2, X³ is a piperazylene group.

X^{a} may be the same as or different from X^{b}, and X^{a} is preferably the same group as X^{b}.

R^{2a} and R^{2b} are each independently an alkylene group or an oxydialkylene group each having not more than 8 carbon atoms, preferably each independently an alkylene group having not more than 8 carbon atoms.

The alkylene group having not more than 8 carbon atoms may be linear or branched, preferably linear. The number of carbons contained in the alkylene group is preferably not more than 6, most preferably not more than 4. Specific examples of the alkylene group having not more than 8 carbon atoms include methylene group, ethylene group, trimethylene group, isopropylene group, tetramethylene group, isobutylene group, pentamethylene group, hexamethylene group, heptamethylene group, octamethylene group, and the like. It is preferably a methylene group, an ethylene group, a trimethylene group, or a tetramethylene group, most preferably an ethylene group.

In the present specification, the "oxydialkylene group having not more than 8 carbon atoms" means an alkylene group via an ether bond (alkylene-O-alkylene, or "alkyleneoxyalkylene group"), wherein the total carbon number of the two alkylene groups present is 8 or below. The two alkylene groups present may be the same or different, preferably the same. Specific examples of the oxydialkylene group having not more than 8 carbon atoms include oxydimethylene group, oxydiethylene group, oxydi(trimethylene) group (i.e., trimethyleneoxytrimethylene group), oxydi(tetramethylene) group (i.e., tetramethyleneoxytetramethylene group), and the like. It is preferably an oxydimethylene group, an oxydiethylene group, or an oxydi(trimethylene) group, most preferably an oxydiethylene group.

R^{2a} may be the same as or different from R^{2b}, and R^{2a} is preferably the same group as R^{2b}.

Y^{a} and Y^{b} are each independently an ester bond, an amide bond, a carbamate bond, an ether bond or a urea bond, preferably each independently an ester bond, an amide bond or a carbamate bond, more preferably each independently an ester bond or an amide bond, most preferably each an ester bond. The direction of the bond of Y^{a} and Y^{b} is not limited. When Y^{a} and Y^{b} are ester bonds, the structure of -Z^{a}-CO-O-R^{2a}- or -Z^{b}-CO-OR^{2b}- is preferably shown.

Y^{a} may be the same as or different from Y^{b}, and Y^{a} is preferably the same group as Y^{b}.

Z^{a} and Z^{b} are each independently a divalent group derived from an aromatic compound having 3 to 16 carbon atoms and at least one aromatic ring, and optionally having a hetero atom. The number of carbons contained in the aromatic compound is preferably 6 to 12, most preferably 6 to 7. The aromatic ring contained in the aromatic compound is preferably one.

As the kind of the aromatic ring contained in the aromatic compound having 3 to 16 carbon atoms, benzene ring, naphthalene ring, anthracene ring can be mentioned for aromatic hydrocarbon ring, and imidazole ring, pyrazole ring, oxazole ring, isoxazole ring, thiazole ring, isothiazole ring, triazine ring, pyrrole ring, furanthiophene ring, pyrimidine ring, pyridazine ring, pyrazine ring, pyridine ring, purine ring, pteridine ring, benzimidazole ring, indole ring, benzofuran ring, quinazoline ring, phthalazine ring, quinoline ring, isoquinoline ring, coumarin ring, chromone ring, benzodiazepine ring, phenoxathiine ring, phenothiazine ring, acridine ring, and the like can be mentioned for aromatic heterocycle. It is preferably a benzene ring, a naphthalene ring, or an anthracene ring, most preferably a benzene ring.

The aromatic ring may have a substituent. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like.

A preferred specific structure of Z^{a} and Z^{b} is Z¹. 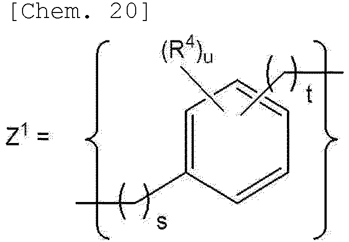 wherein s is an integer of 0 to 3, t is an integer of 0 to 3, u is an integer of 0 to 4, and R⁴ in the number of u are each independently a substituent.

The s in Z¹ is preferably an integer of 0 to 1, more preferably 0.

The t in Z¹ is preferably an integer of 0 to 2, more preferably 1.

The u in Z¹ is preferably an integer of 0 to 2, more preferably an integer of 0 to 1.

The R⁴ in Z¹ is a substituent of an aromatic ring (benzene ring) contained in the aromatic compound having 3 to 16 carbon atoms which does not inhibit the reaction in the synthesis process of the ionic lipid. Examples of the substituent include acyl group having 2 to 4 carbon atoms, alkoxycarbonyl group having 2 to 4 carbon atoms, carbamoyl group having 2 to 4 carbon atoms, acyloxy group having 2 to 4 carbon atoms, acylamino group having 2 to 4 carbon atoms, alkoxycarbonylamino group having 2 to 4 carbon atoms, fluorine atom, chlorine atom, bromine atom, iodine atom, alkylsulfanyl group having 1 to 4 carbon atoms, alkylsulfonyl group having 1 to 4 carbon atoms, arylsulfonyl group having 6 to 10 carbon atoms, nitro group, trifluoromethyl group, cyano group, alkyl group having 1 to 4 carbon atoms, ureido group having 1 to 4 carbon atoms, alkoxy group having 1 to 4 carbon atoms, aryl group having 6 to 10 carbon atoms, aryloxy group having 6 to 10 carbon atoms, and the like. Preferred examples include acetyl group, methoxycarbonyl group, methylcarbamoyl group, acetoxy group, acetamido group, methoxycarbonylamino group, fluorine atom, chlorine atom, bromine atom, iodine atom, methylsulfanyl group, phenylsulfonyl group, nitro group, trifluoromethyl group, cyano group, methyl group, ethyl group, propyl group, isopropyl group, tert-butyl group, ureido group, methoxy group, ethoxy group, propoxy group, isopropoxy group, tert-butoxy group, phenyl group, phenoxy group, and the like. When R⁴ is present in plurality, each R⁴ may be the same or different.

Z^{a} may be the same as or different from Z^{b}, and Z^{a} is preferably the same group as Z^{b}.

n^{a} and n^{b} are each independently 0 or 1.

n^{a} may be the same as or different from n^{b}, and n^{a} is preferably the same as n^{b}.

R^{3a} and R^{3b} are each independently a group such that R^{3a}-COO- and R^{3b}-COO- are each a residue derived from a reaction of a hydroxyl group of vitamin E, and succinic anhydride or glutaric anhydride.

A residue derived from a reaction product of vitamin E, and succinic anhydride or glutaric anhydride is a group with a structure in which a hydroxyl group of vitamin E having the hydroxyl group is substituted with *-O-CO-CH₂-CH₂- or *-O-CO-CH₂-CH₂-CH₂-. * shows a bonding position with vitamin E.

As vitamin E, α-tocopherol, β-tocopherol, γ-tocopherol, δ-tocopherol, α-tocotrienol, β-tocotrienol, γ-tocotrienol, and δ-tocotrienol can be specifically mentioned. It is preferably α-tocopherol, β-tocopherol, α-tocotrienol, or β-tocotrienol, most preferably α-tocopherol.

In one embodiment of the present invention, R¹³ is the same as R^{1b}, X^{a} is the same as X^{b}, R^{2a} is the same as R^{2b}, Y^{a} is the same as Y^{b}, Z^{a} is the same as Z^{b}, and R^{3a} is the same as R^{3b}.

Preferred examples of the vitamin E derivative represented by the formula (3) incluce the following compounds.

### [Vitamin E derivative (3-1)]

Compound (3) wherein
R^{1a} and R^{1b} are each independently an alkylene group having 1 to 6 carbon atoms (e.g., methylene group, ethylene group);
X^{a} and X^{b} are each independently an acyclic alkyl tertiary amino group having 1 to 6 carbon atoms and one tertiary amino group (e.g., -N(CH₃)-), or a cyclic alkylene tertiary amino group having 2 to 5 carbon atoms and 1 to 2 tertiary amino groups (e.g., piperidylene group);
R^{2a} and R^{2b} are each independently an alkylene group having not more than 8 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 3 to 16 carbon atoms and at least one aromatic ring, and optionally having a hetero atom (e.g., -C₆H₄-CH₂-, -CH₂-C₆H₄-CH₂-);
n^{a} and n^{b} are each independently 0 or 1; and
R^{3a} and R^{3b} are each independently a group such that R^{3a}-COO- and R^{3b}-COO- are each a residue derived from a reaction of a hydroxyl group of vitamin E, and succinic anhydride or glutaric anhydride.

### [Vitamin E derivative (3-2)]

Compound (3) wherein
R^{1a} and R^{1b} are each independently an alkylene group having 1 to 4 carbon atoms (e.g., methylene group, ethylene group);
X^{a} and X^{b} are each independently an acyclic alkyl tertiary amino group having 1 to 3 carbon atoms and one tertiary amino group (e.g., -N(CH₃)-), or a cyclic alkylene tertiary amino group having 2 to 5 carbon atoms and one tertiary amino group (e.g., piperidylene group);
R^{2a} and R^{2b} are each independently an alkylene group having not more than 6 carbon atoms (e.g., methylene group, ethylene group, trimethylene group);
Y^{a} and Y^{b} are each independently an ester bond or an amide bond;
Z^{a} and Z^{b} are each independently a divalent group derived from an aromatic compound having 6 to 12 carbon atoms and one aromatic ring, and optionally having a hetero atom (e.g., - C₆H₄-CH₂-, -CH₂-C₆H₄-CH₂-);
n^{a} and n^{b} are each independently 0 or 1; and
R^{3a} and R^{3b} are each independently a group such that R^{3a}-COO- and R^{3b}-COO- are each a residue derived from a reaction of a hydroxyl group of vitamin E, and succinic anhydride or glutaric anhydride.

### [Vitamin E derivative (3-3)]

Compound (3) wherein
R^{1a} and R^{1b} are each independently an alkylene group having 1 to 2 carbon atoms (i.e., methylene group or ethylene group);
X^{a} and X^{b} are each independently X¹: 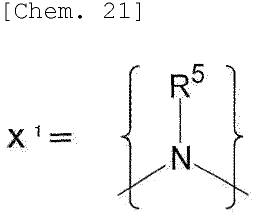 wherein R⁵ is an alkyl group having 1 to 3 carbon atoms (e.g., methyl group), or X²: 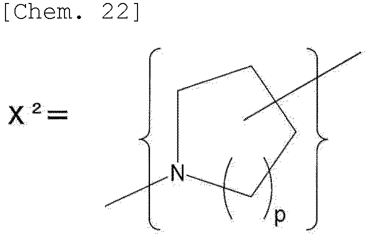 wherein p is 1 or 2;
R^{2a} and R^{2b} are each independently alkylene group having not more than 4 carbon atoms (e.g., methylene group, ethylene group, trimethylene group);
Y^{a} and Y^{b} are each independently an ester bond or an amide bond;
Z^{a} and Z^{b} are each independently Z¹: 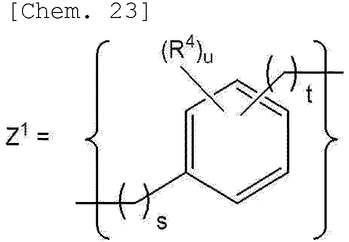 wherein s is an integer of 0 to 1, t is an integer of 0 to 2, u is an integer of 0 to 2 (preferably 0), and R⁴ in the number of u are each independently a substituent;
n^{a} and n^{b} are each independently 0 or 1; and
R^{3a} and R^{3b} are each independently a group such that R^{3a}-COO- and R^{3b}-COO- are each a residue derived from a reaction of a hydroxyl group of vitamin E, and succinic anhydride or glutaric anhydride.

Specific examples of the vitamin E derivative of the formula (3) include the following B-2-5, TS-C4E, TS-P3C1, TS-P4C1, TS-P4C2, E-Ph-P4C2, TS-P4C3, TS-P4C4, TG-C3M, and TSamide-C3M. Also, these derivatives in which the α-tocopherol residue is substituted by β-tocopherol, α-tocotrienol, or β-tocotrienol residue can be mentioned.

**[Table 2-1]**

| vitamin E derivative (3) | structure |
|---|---|
| B-2-5 (or SS-E) | 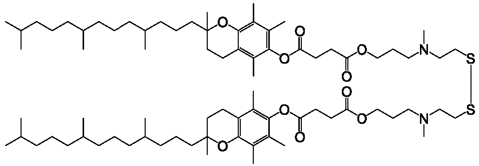 |
| TS-C4E | 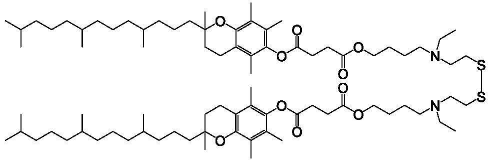 |
| TS-P3C1 | 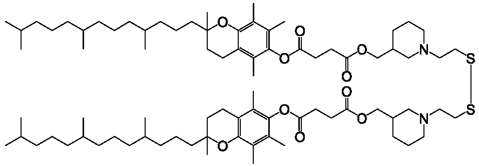 |
| TS-P4C1 | 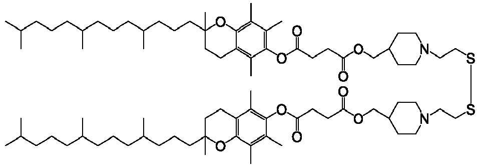 |
| TS-P4C2 (or SS-EC) | 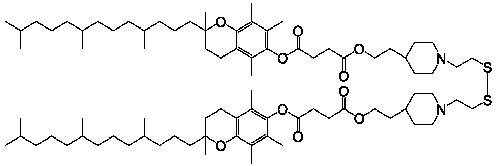 |
| E-Ph-P4C2 (or SS-EP) | 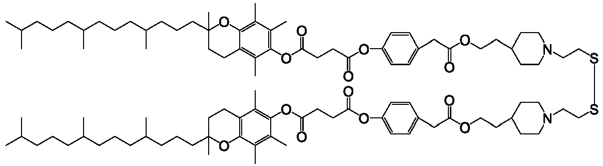 |

**[Table 2-2]**

| vitamin E derivative (3) | structure |
|---|---|
| TS-P4C3 | 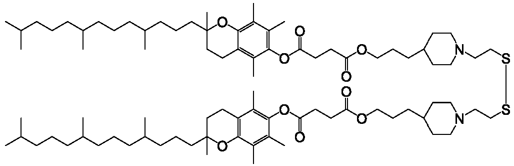 |
| TS-P4C4 | 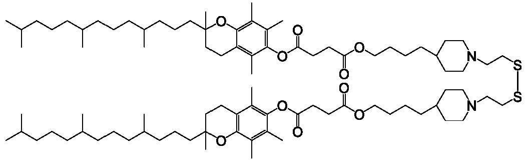 |
| TG-C3M | 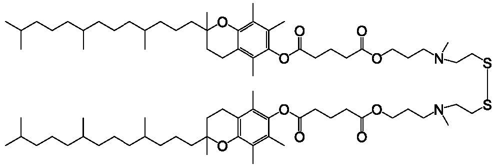 |
| TSamide-C3M | 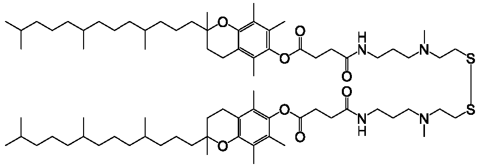 |

Among the specific examples of the vitamin E derivative of the formula (3), SS-E and SS-EC are preferred. That is, the vitamin E derivative of the formula (3) is preferably an ionic lipid represented by the following formula: 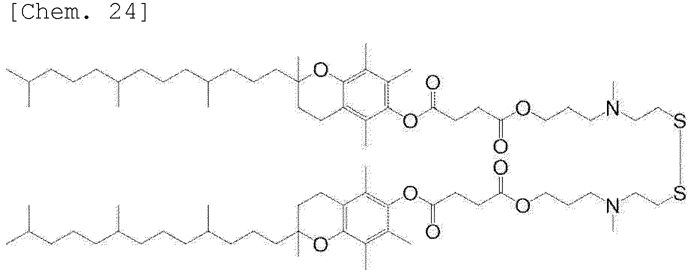 or

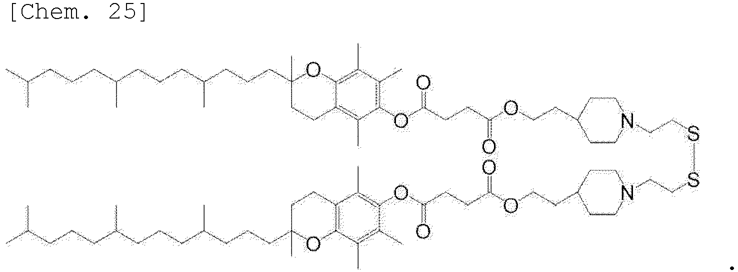

Other specific preferred examples of the vitamin E derivative of the formula (3) include compounds of SS-E and SS-EC in which the α-tocopherol residue is substituted by β-tocopherol, α-tocotrienol, or β-tocotrienol residue. Therefore, in one preferred embodiment, the vitamin E derivative of the formula (3) is a compound represented by the formula (4): 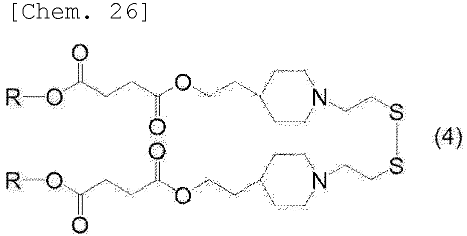 (in the formula (4),
R is a group such that RCOO(CH₂)₂COO- is a residue derived from a reaction of a hydroxyl group of α-tocopherol, β-tocopherol, α-tocotrienol, or β-tocotrienol and succinic anhydride), or the formula (5): 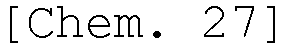 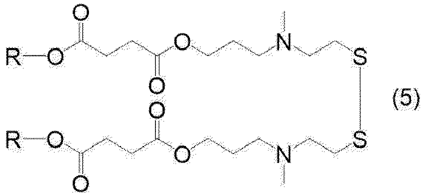 (in the formula (5),
R is a group such that RCOO(CH₂)₂COO- is a residue derived from a reaction of a hydroxyl group of α-tocopherol, β-tocopherol, α-tocotrienol, or β-tocotrienol and succinic anhydride).

The vitamin E derivative of the formula (3) can be produced by a known method (e.g., US 9708628 B2, WO2019/188867 A1).

The total amount of vitamin E and/or a derivative thereof in the lipid nanoparticles of the present invention is preferably 4.5 to 35 mol%, more preferably 10 to 30 mol%, with respect to the total of ionic lipid (1), vitamin E and/or a derivative thereof, phospholipid and cholesterol, from the aspects of efficiency of nucleic acid encapsulation, efficiency of intracellular release of nucleic acid, and stability of the lipid nanoparticles. In addition, in the lipid nanoparticles of the present invention, either vitamin E or a vitamin E derivative alone may be used or these may be used in combination. Preferably, either one can be preferably used.

### Phospholipid

The lipid nanoparticles of the present invention include phospholipid. Only one kind of phospholipid may be used, or phospholipid or other phospholipid may be used in combination. In this case, the ratio of other phospholipids to the phospholipid is not particularly limited as long as the effect of the present invention can be expressed.

Specific examples of the phospholipid include, 1,2-diacyl-sn-glycero-3-phosphocholine (PC), 1,2-diacyl-sn-glycero-3-phosphoethanolamine (PE), lyso forms of these, and the like. In the present specification, phospholipids may be sometimes indicated with the abbreviations thereof. For example, 1,2-diacyl-sn-glycero-3-phosphocholine is sometimes indicated as PC, and 1,2-didecanoyl-sn-glycero-3-phosphocholine is sometimes indicated as DDPC.

Specific examples of 1,2-diacyl-sn-glycero-3-phosphocholine (PC) include 1,2-didecanoyl-sn-glycero-3-phosphocholine (DDPC), 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilinoleoyl-sn-glycero-3-phosphocholine (DLoPC), 1,2-diarachidoyl-sn-glycero-3-phosphocholine (DAPC) 1,2-dieicosenoyl-sn-glycero-3-phosphocholine (DEiPC), 1,2-dibehenoyl-sn-glycero-3-phosphocholine (DBPC), 1,2-dierucoyl-sn-glycero-3-phosphocholine (DEPC), 1-myristoyl-2-palmitoyl-sn-glycero-3-phosphocholine (MPPC), 1-myristoyl-2-stearoyl-sn-glycero-3-phosphocholine (MSPC), 1-palmitoyl-2-myristoyl-sn-glycero-3-phosphocholine (PMPC), 1-palmitoyl-2-stearoyl-sn-glycero-3-phosphocholine (PSPC), 1-palmitoyl-2-oleoyl-sn-glycero-3-phosphocholine (POPC), 1-stearoyl-2-oleoyl-sn-glycero-3-phosphocholine (SOPC).

Specific examples of 1,2-diacyl-sn-glycero-3-phosphoethanolamine (PE) include 1,2-didecanoyl-sn-glycero-3-phosphoethanolamine (DDPE), 1,2-dilauroyl-sn-glycero-3-phosphoethanolamine (DLPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), 1,2-dioleoyl-sn-glycero-3-phosphoethanolamine (DOPE), 1,2-dilinoleoyl-sn-glycero-3-phosphoethanolamine (DLoPE), 1,2-dierucoyl-sn-glycero-3-phosphoethanolamine (DEPE), 1-myristoyl-2-palmitoyl-sn-glycero-3-phosphoethanolamine (MPPE), 1-myristoyl-2-stearoyl-sn-glycero-3-phosphoethanolamine (MSPE), 1-palmitoyl-2-myristoyl-sn-glycero-3-phosphoethanolamine (PMPE), 1-palmitoyl-2-stearoyl-sn-glycero-3-phosphoethanolamine (PSPE), 1-palmitoyl-2-oleoyl-sn-glycero-3-phosphoethanolamine (POPE), 1-stearoyl-2-oleoyl-sn-glycero-3-phosphoethanolamine (SOPE).

The phospholipid is not particularly limited and is preferably at least one selected from the group consisting of DOPC, DSPC, POPC, DOPE, and POPE, more preferably at least one of DOPC and DOPE. These phospholipids have similar acyl chain structures and are therefore considered to exhibit similar effects.

The amount of the phospholipid in the lipid nanoparticle of the present invention is preferably 5 to 35 mol%, more preferably 6 to 30 mol%, with respect to the total of ionic lipid (1), vitamin E and/or a derivative thereof, phospholipid and cholesterol, from the aspects of efficiency of nucleic acid encapsulation, efficiency of intracellular release of nucleic acid, and stability of the lipid nanoparticles.

### Cholesterol

The lipid nanoparticles of the present invention include cholesterol. The amount of cholesterol in the lipid nanoparticle of the present invention is preferably 10 to 50 mol%, more preferably 30 to 45 mol%, further preferably 30 to 40 mol%, with respect to the total of ionic lipid (1), vitamin E and/or a derivative thereof, phospholipid and cholesterol, from the aspects of efficiency of nucleic acid encapsulation, efficiency of intracellular release of nucleic acid, and stability of the lipid nanoparticles.

### PEG lipid

The lipid nanoparticles of the present invention include PEG lipid. The PEG lipid is not particularly limited and is preferably a compound represented by the formula (7).

A dimyristoylglycerol PEG represented by the formula (7):

CH₂(OR⁶)-CH(OR⁷)-CH₂(OR⁸) (7)

(in the formula (7),
any two of R⁶, R⁷, and R⁸ are myristoyl groups, and the remaining one is an alkyl group having 1 to 6 carbon atoms connected via a polyethylene glycol (PEG) chain with a number average molecular weight of 1,000 to 3,000)
(sometimes to be abbreviated as "dimyristoylglycerol PEG (7)" in the present specification) is included.

The number average molecular weight of the PEG chain in the formula (7) is 1,000 to 3,000, preferably 1,500 to 2,500. The number average molecular weight of PEG used to form the PEG chain can be measured by gel permeation chromatography (GPC).

The alkyl group having 1 to 6 carbon atoms may be linear, branched or cyclic. The alkyl group preferably has a carbon number of 1 to 3. Specific examples of the alkyl group having 1 to 6 carbon atoms include methyl group, ethyl group, propyl group, isopropyl group, n-butyl group, sec-butyl group, isobutyl group, tert-butyl group, pentyl group, isopentyl group, neopentyl group, tert-pentyl group, 1,2-dimethylpropyl group, 2-methylbutyl group, 2-methylpentyl group, 3-methylpentyl group, 2,2-dimethylbutyl group, 2,3-dimethylbutyl group, cyclohexyl group, and the like. It is preferably a methyl group.

The amount of the PEG lipid in the lipid nanoparticle of the present invention is preferably 0.5 to 4 mol%, more preferably 1 to 3 mol%, most preferably 1.5 mol%, with respect to the total of ionic lipid (1), vitamin E and/or a derivative thereof, phospholipid and cholesterol, from the aspects of efficiency of nucleic acid encapsulation, efficiency of intracellular release of nucleic acid, and stability of the lipid nanoparticles.

### Other lipid

The lipid nanoparticles of the present invention may contain lipid other than an ionic lipid represented by the formula (1), vitamin E and/or a derivative thereof, phospholipid, cholesterol, and PEG lipid (hereinafter sometimes to be referred to as "other lipid"). Examples of other lipid include sterols other than cholesterol, PEG phospholipid, and the like.

The amount of said other lipid in the lipid nanoparticles of the present invention is preferably 0 to 50 mol%, more preferably 0 to 30 mol%, further preferably 0 to 10 mol%, with respect to the total amount of lipids in the lipid nanoparticle. As used herein, "the total amount of lipids in the lipid nanoparticle" when, for example, the lipid nanoparticles contain ionic lipid represented by the formula (1), vitamin E and/or a derivative thereof, phospholipid, cholesterol, PEG lipid, and other lipid as constituent components means "total amount of the ionic lipid represented by the formula (1), vitamin E and/or a derivative thereof, phospholipid, cholesterol, PEG lipid, and other lipid". In the present specification, the "amount of B (mol%) with respect to A" means the "100 × amount of B (mol)/amount of A (mol)". For example, the "amount of other lipid (mol%) with respect to the total amount of lipids" means the "100 × amount of other lipid (mol)/total amount of lipid (mol)".

In the present invention, it is most preferred that no other lipids are used. That is, the lipids constituting the lipid nanoparticles of the present invention most preferably consist of an ionic lipid represented by the formula (1), vitamin E and/or a derivative thereof, phospholipid, cholesterol, and PEG lipid.

### Lipid composition

As the lipid composition of the lipid nanoparticles of the present invention, the molar ratio of ionic lipid
(1):vitamin E and/or a derivative thereof (excluding vitamin derivatives of the formula (3)):phospholipid:cholesterol:PEG lipid is preferably 30 to 55:4.5 to 20:5 to 15:20 to 50:1 to 3, more preferably 40 to 50:10 to 20:5 to 10:30 to 40:1 to 2, most preferably 43.8:16.7:6.2:33.3:1.5.

On the other hand, when a vitamin derivative of the formula (3) is used, the optimal composition of the lipid nanoparticles in one embodiment of the present invention is the molar ratio of ionic lipid (1):vitamin E derivative
(3):phospholipid:cholesterol:PEG lipid of preferably 20 to 55:4.5 to 35:5 to 35:10 to 50:1 to 3, more preferably 20 to 50:10 to 30:7.5 to 30:10 to 45:1 to 3, further preferably 25 to 40:10 to 30:7.5 to 10:30 to 45:1 to 3, most preferably **32.5:20:7.5:40:1.5.**

When a vitamin derivative of the formula (3) is used, the optimal composition of the lipid nanoparticles in another embodiment of the present invention is the molar ratio of ionic lipid (1):vitamin E derivative (3):phospholipid:cholesterol:PEG lipid of preferably 20 to 55:4.5 to 35:5 to 35:10 to 50:1 to 3, more preferably 20 to 50:10 to 30:7.5 to 30:10 to 40:1 to 3, most preferably **32.5:20:7.5:40:1.5.**

### Production method of lipid nanoparticles

The lipid nanoparticles of the present invention can be produced by through a vitamin addition step of adding vitamin E and/or a derivative thereof to a ionic lipid represented by the formula (1), phospholipid, cholesterol, and PEG lipid. Specifically, the lipid nanoparticles can be produced by preparing a lipid solution by a method including adding a solution of vitamin E and/or a derivative thereof dispersed in a suitable dispersion medium (e.g., aqueous dispersion medium, alcoholic dispersion medium) to a solution of a lipid material containing ionic lipid (1), phospholipid, cholesterol, and PEG lipid dispersed in a suitable dispersion medium (e.g., aqueous dispersion medium, alcoholic dispersion medium), and performing an operation to induce organization as necessary (sometimes referred to as "pre-addition method" in the present specification).

Examples of the "operation to induce organization" for producing the lipid nanoparticles of the present invention in the pre-addition method include methods known per se such as ethanol dilution method using a microchannel or vortex, simple hydration method, sonication, heating, vortex, ether injecting method, French press method, cholic acid method, Ca²⁺ fusion method, freeze-thaw method, reversed-phase evaporation method, and the like. It is preferably an ethanol dilution method using a microchannel or vortex, further preferably an ethanol dilution method using a microchannel. In the ethanol dilution method using a microchannel, for example, a dispersion containing lipid nanoparticles encapsulating a desired encapsulated substance (preferably, nucleic acid) can be produced by mixing an acidic buffer containing the encapsulated substance and an ethanol solution of a lipid prepared as mentioned above, by using NanoAssemblr (registered trademark) (Precision NanoSystems). The dispersion produced by this method contains the lipid nanoparticles and a dispersion medium (acidic buffer and ethanol). The dispersion medium (particularly ethanol) can be removed, the dispersion medium (particularly buffer) can be exchanged, and the like by operations such as ultrafiltration, dialysis, dilution, and the like.

As another form for producing the lipid nanoparticles of the present invention encapsulating the desired encapsulation substance (preferably, nucleic acid), the lipid nanoparticles can also be produced by a method in which a lipid solution containing lipid materials including ionic lipid (1), phospholipid, cholesterol, and PEG lipid dispersed in a suitable dispersion medium (e.g., aqueous dispersion medium, alcoholic dispersion medium) and an acidic buffer solution containing the encapsulated substance are used to prepare a dispersion liquid, and then vitamin E or a vitamin E derivative is added during steps such as removal of the dispersion medium (particularly ethanol), exchange of the dispersion medium (particularly buffer), and the like by operations such as ultrafiltration, dialysis, dilution, and the like (sometimes referred to as the "post-addition method" in the present specification).

Examples of the method for preparing a dispersion containing lipid nanoparticles by the post-addition method include methods known per se such as ethanol dilution method using a microchannel or vortex, simple hydration method, sonication, heating, vortex, ether injecting method, French press method, cholic acid method, Ca²⁺ fusion method, freeze-thaw method, reversed-phase evaporation method, and the like. It is preferably an ethanol dilution method using a microchannel or vortex, further preferably an ethanol dilution method using a microchannel. For example, when the lipid nanoparticles of the present invention are produced by adding vitamin E or a vitamin E derivative to a dispersion prepared by an ethanol dilution method using a microchannel, the ethanol solution of vitamin E or a vitamin E derivative may be added directly in any step after the preparation of the dispersion. However, it is preferred to add the ethanol solution at least in one of the steps immediately after the preparation of the dispersion, during the dilution operation of the dispersion medium, or during the exchange of the dispersion medium (particularly buffer) by ultrafiltration or dialysis.

### Method for obtaining acquired immunity by nucleic acid delivery

The present invention also provides a method for imparting acquired immunity to a subject by delivering a desired encapsulated substance (preferably, nucleic acid) to the subject, including administering the lipid nanoparticles of the present invention encapsulating the encapsulated substance to the subject. In this method, the lipid nanoparticles are preferably subcutaneously or intramuscularly administered to the subject.

The encapsulation substance to be encapsulated in the lipid nanoparticles of the present invention is not particularly limited as long as the desired acquired immunity is imparted by delivery into the cells of the subject. Examples thereof include protein or fragment thereof, nucleic acid, and the like. Examples of the nucleic acid include, but are not limited to, DNA, RNA, chimera nucleic acid of RNA, DNA/RNA hybrid, and the like. While any single-stranded to triple-stranded nucleic acid can be used, it is preferably single-stranded or double-stranded. The nucleic acid may be a nucleotide having N-glycoside of purine or pyrimidine base, an oligomer having a non-nucleotide backbone (e.g., commercially available peptide nucleic acid (PNA) etc.), or an oligomer containing a special bond (said oligomer containing a nucleotide having a configuration permitting base pairing or attachment of base, which are found in DNA and RNA) and the like.

Furthermore, the nucleic acid may be, for example, a nucleic acid added with known modification, a nucleic acid with a label known in the field, a nucleic acid with a cap, a methylated nucleic acid, a nucleic acid with one or more natural nucleotides substituted by an analog, a nucleic acid with modified nucleotide, a nucleic acid having a non-charge bond (e.g., methylphosphonate, phosphotriester, phosphoramidate, carbamate, and the like), a nucleic acid having a charged bond or sulfur-containing bond (e.g., phosphorothioate, phosphorodithioate, and the like), a nucleic acid having a side chain group such as protein (e.g., nuclease, nuclease inhibitor, toxin, antibody, signal peptide, poly-L-lysine, and the like), sugar (e.g., monosaccharide and the like), and the like, a nucleic acid containing an intercalating compound (e.g., acridine, psoralen, and the like), a nucleic acid containing a chelate compound (e.g., metal, radioactive metal, boron, oxidative metal, and the like), a nucleic acid containing an alkylating agent, or a nucleic acid containing a modified bond (e.g., α anomer-type nucleic acid and the like).

The type of the DNA that can be used in the present invention is not particularly limited, and can be selected as appropriate according to the purpose of use. Examples of the DNA include plasmid DNA, cDNA, antisense DNA, chromosomal DNA, PAC, BAC, CpG-oligo, and the like. Preferred are plasmid DNA, cDNA and antisense DNA, and more preferred is plasmid DNA. A circular DNA such as plasmid DNA and the like can be digested as appropriate with a restriction enzyme and the like, and also used as a linear DNA.

The type of the RNA that can be used in the present invention is not particularly limited, and can be selected as appropriate according to the purpose of use. Examples of the RNA include messenger RNA (mRNA), pre-mRNA, single-stranded RNA genome, double-stranded RNA genome, siRNA, miRNA, shRNA, antisense RNA, RNA replicon, transfer RNA, ribosomal RNA, and the like. Preferred are mRNA, pre-mRNA, siRNA, miRNA, shRNA, antisense RNA, and RNA replicon.

The nucleic acid used in the present invention is preferably purified by a method generally used by those of ordinary skill in the art.

The nucleic acid to be encapsulated in the lipid nanoparticles of the present invention is preferably one having a preventive and/or therapeutic activity against a given disease (prophylactic/therapeutic nucleic acid). Examples of such nucleic acid include nucleic acids used for so-called gene therapy and vaccine, and the like. Nucleic acids used for vaccine applications include nucleic acids (expression vectors, (pre)mRNA) encoding antigenic proteins or peptides that are presented on the surface of pathogenic cells (including pathogenic bacteria and cancer cells) of a target disease or viruses. When the lipid nanoparticles of the present invention encapsulating the nucleic acid are delivered into cells, the nucleic acid is (transcribed and) translated to generate an antigen protein or peptide, which is recognized by immune cells such as B cells and T cells, and neutralizing antibodies and killer T cells specific to the antigen protein or peptide are produced, thus imparting acquired immunity. Nucleic acids encoding such antigen proteins or peptides can be appropriately selected and used by those skilled in the art according to the target diseases. For example, in the case of a vaccine for COVID-19, DNA or mRNA encoding the spike protein derived from the SARS-CoV-2 virus or a fragment thereof can be appropriately designed based on the genome sequence information of the virus.

The particle size of the lipid nanoparticle encapsulating a nucleic acid is not particularly limited, and is preferably 10 nm to 500 nm, more preferably 30 nm to 300 nm. The particle size can be measured by using a particle size distribution measuring device such as Zetasizer Nano (Malvern) or the like. The particle size of the lipid nanoparticle encapsulating a nucleic acid can be appropriately adjusted by the production method thereof.

The surface potential (zeta potential) of lipid nanoparticles used in nucleic acid vaccines that can efficiently enhance acquired immunity is preferably -15 to +15 mV, more preferably -10 to 10 mV. In conventional transgene, particles with positively charged surfaces have been mainly used. This is useful as a method for promoting electrostatic interactions with heparin sulfate on the negatively-charged cell surface to enhance uptake into cells. However, the positive surface potential may suppress (a) nucleic acid release from the carrier due to the interaction with the delivered nucleic acid in the cell, or (b) protein synthesis due to the interaction between mRNA and the delivered nucleic acid. This problem can be solved by adjusting the surface potential (zeta potential) to fall within the above-mentioned range. The surface potential (zeta potential) can be measured using a zeta potential measuring apparatus such as Zetasizer Nano or the like. The surface potential (zeta potential) of the lipid nanoparticles can be adjusted by the composition of the constituent components of the lipid nanoparticles.

By administering the lipid nanoparticles of the present invention encapsulating nucleic acid to a living body, the lipid nanoparticles are taken into cells, and the nucleic acid encapsulated in the lipid nanoparticles is delivered to cells in the living body and functions to afford acquired immunity. The subjects to which the lipid nanoparticles can be administered are not particularly limited, and examples thereof include cells of mammals (e.g., human, monkey, mouse, rat, hamster, bovine, etc.), birds (e.g., chicken, ostrich, etc.), amphibia (e.g., frog etc.), fish (e.g., zebrafish, rice-fish, etc.), and the like. The subjects to which the lipid nanoparticles are introduced are preferably cells of humans or other mammals.

The administration method of the lipid nanoparticles encapsulating a nucleic acid to a subject is not particularly limited as long as the lipid nanoparticles can deliver nucleic acid to spleen cells, and an administration method known per se (e.g., oral administration, parenteral administration (e.g., transnasal administration, intravenous administration, intramuscular administration, topical administration, transdermal administration, subcutaneous administration, intraperitoneal administration, spray, etc.), etc.) can be appropriately selected. As the administration method, intramuscular administration and subcutaneous administration are preferred. The dose of the lipid nanoparticles can be appropriately selected in consideration of the kind of the subject of administration, administration method, and the like.

The lipid nanoparticles of the present invention may be used as they are, or mixed with a pharmaceutically acceptable carrier and can be produced as an oral agent (e.g., tablet, capsule agent, etc.) or a parenteral agent (e.g., nasal preparation, injection, inhalant, etc.), preferably a parenteral agent (more preferably, injection).

As the pharmaceutically acceptable carrier, those conventionally used as formulation materials are used. For example, in solid preparations, excipient, lubricant, binder, disintegrant, and the like are used, and in liquid preparations, solvent, solubilizing agent, suspending agent, isotonicity agent, buffering agent, soothing agent, and the like are used. Where necessary, formulation additives such as antiseptic, antioxidant, colorant, sweetening agent, and the like can also be used.

For example, in the case of nasal preparations, they are used in the form of nasal drops or sprays.

### [Example]

The present invention is explained in more detail in the following by using Examples and the like; however, the present invention is not limited in any way by the following Examples and the like.

In the following Examples and the like, ionic lipid (1) and an ionic lipid having vitamin E skeleton are shown by the names listed in the aforementioned Tables. The abbreviations used in the following Examples and the like each mean the following.
Chol: cholesterol
DOPC: 1,2-dioleoyl-sn-glycero-3-phosphocholine
DOPE: 1,2-dioleoyl-sn-glycero-3-phosphoethanolamine
DMG-PEG2000: 1,2-dimyristoyl-rac-glycerol, methoxypolyethylene glycol (number average molecular weight (Mn) of PEG: 2000)
VE: D-α-tocopherol
TS: D-α-tocopherol succinate
MES: 2-morpholinoethanesulfonic acid
PBS: phosphate buffered saline
OVA-mRNA: mRNA encoding ovalbumin
Luc-mRNA: mRNA encoding luciferase
CFSE: carboxyfluorescein diacetate succinimidyl ester

The mol% of lipid composition is mol% relative to the total of ionic lipid (1), vitamin E and/or a derivative thereof, phospholipid, and cholesterol.

### [Production Example 1] Preparation of mRNA-encapsulating LNP by ethanol dilution method - 1 (post-addition method)

### (1) Preparation of ethanolic solution of lipids

Each lipid was dissolved in ethanol to give 10 mM SS-OP, 5 mM SS-EC, 10 mM VE, 10 mM TS, 10 mM DOPC, 10 mM DOPE, 10 mM Chol, or 1 mM DMG-PEG2000. From these solutions, lipids were selected and mixed in certain ratios such that the total mole amount of SS-OP, DOPC, and Chol was 1000 nmol, and 1 mM DMG-PEG2000 ethanol solution was added thereto to achieve the desired ratio. Finally, ethanol was added to a total amount of 450 µL to prepare an ethanol solution of the lipids.

### (2) Preparation of acidic buffer solution of nucleic acid

The acidic buffer solution of nucleic acid was prepared by mixing mRNA encoding luciferase or ovalbumin (Luc-mRNA or OVA-mRNA) at a concentration of 0.0067 µg/µL with 20 mM acidic malic acid buffer (pH 3.0) containing 30 mM NaCl.

### (3) Preparation of dispersion by ethanol dilution method

Using NanoAssemblr (registered trademark) ultra-highspeed nanomedicine production device (manufactured by Precision NanoSystems), 1200 µL of nucleic acid acidic buffer solution and 300 µL of lipid ethanol solution were mixed at flow rates of 3 mL/min and 1 mL/min, respectively, and 1 mL of dispersion was collected.

### (3-1) Addition of VE to dispersion (post-addition method 1)

After adding 10 mM VE solution to the collected dispersion at any ratio, the mixture was diluted with 3 mL of 20 mM MES buffer (pH 6.5) and transferred to Amicon Ultra 4 (Millipore). The transferred LNP solution was concentrated to about 500 µL by ultrafiltration under centrifugation conditions (25°C, 1000 g, about 10 min). The obtained concentrate was diluted with PBS to 4 mL, and concentrated again to about 200 µL by ultrafiltration under centrifugation conditions (25°C, 1000 g, about 10 min). Finally, the volume was increased by diluting with PBS to a nucleic acid concentration of 10 µg/mL.

### (3-2) Addition of VE to LNP after dilution (post-addition method 2)

The collected dispersion was diluted with 3 mL of 20 mM MES buffer (pH 6.5), 10 mM VE solution was added thereto at any ratio, and the mixture was transferred to Amicon Ultra 4 (Millipore). The transferred LNP solution was concentrated to about 500 µL by ultrafiltration under centrifugation conditions (25°C, 1000 g, about 10 min). The obtained concentrate was diluted with PBS to 4 mL, and concentrated again to about 200 µL by ultrafiltration under centrifugation conditions (25°C, 1000 g, about 10 min). Finally, the volume was increased by diluting with PBS to a nucleic acid concentration of 10 µg/mL.

### (3-3) Addition of VE to LNP during ultrafiltration (post-addition method 3)

The collected dispersion was diluted with 3 mL of 20 mM MES buffer (pH 6.5) and transferred to Amicon Ultra 4 (Millipore). The transferred LNP solution was concentrated to about 500 µL by ultrafiltration under centrifugation conditions (25°C, 1000 g, about 10 min). The obtained concentrate was diluted with PBS to 4 mL, 10 mM VE solution was added thereto at any ratio, and the mixture was concentrated again to about 200 µL by ultrafiltration under centrifugation conditions (25°C, 1000 g, about 10 min). Finally, the volume was increased by diluting with PBS to a nucleic acid concentration of 10 µg/mL.

### [Production Example 2] Preparation of mRNA-encapsulating LNP by ethanol dilution method - 2 (pre-addition method)

### (1-1) Preparation of ethanolic solution of lipids

Each lipid was dissolved in ethanol to give 10 mM SS-OP, 5 mM SS-EC, 10 mM VE, 10 mM TS, 10 mM DOPC, 10 mM DOPE, 10 mM Chol, or 1 mM DMG-PEG2000. From these solutions, lipids were selected and mixed in certain ratios such that the total mole amount of SS-OP, DOPC, and Chol was 1000 nmol, 10 mM VE, 10 mM TS or 5 mM SS-EC, and 1 mM DMG-PEG2000 ethanol solution were added thereto to achieve the desired ratio. Finally, ethanol was added to a total amount of 450 µL to prepare an ethanol solution of the lipids.

### (1-2) Preparation of ethanolic solution of lipids

Each lipid was dissolved in ethanol to give 10 mM SS-OP, 5 mM SS-EC, 10 mM DOPC, 10 mM DOPE, 10 mM Chol, or 1 mM DMG-PEG2000. From these solutions, lipids were selected and mixed in certain ratios such that the total mole amount of SS-OP, SS-EC, DOPC or DOPE, and Chol was 1000 nmol, and 1 mM DMG-PEG2000 ethanol solution was added thereto to achieve the desired ratio. Finally, ethanol was added to a total amount of 450 µL to prepare an ethanol solution of the lipids.

### (2) Preparation of acidic buffer solution of nucleic acid

The acidic buffer solution of nucleic acid was prepared by mixing mRNA encoding luciferase or ovalbumin (Luc-mRNA or OVA-mRNA) at a concentration of 0.0067 µg/µL with 20 mM acidic malic acid buffer (pH 3.0) containing 30 mM NaCl.

### (3) Preparation of LNP by ethanol dilution method

Using NanoAssemblr (registered trademark) ultra-highspeed nanomedicine production device (manufactured by Precision NanoSystems), 1200 µL of nucleic acid acidic buffer solution and 300 µL of lipid ethanol solution were mixed at flow rates of 3 mL/min and 1 mL/min, respectively, and 1 mL of dispersion was collected. The collected dispersion was diluted with 3 mL of 20 mM MES buffer (pH 6.5) and transferred to Amicon Ultra 4 (Millipore). The transferred LNP solution was concentrated to about 500 µL by ultrafiltration under centrifugation conditions (25°C, 1000 g, about 10 min). The obtained concentrate was diluted with PBS to 4 mL, and concentrated again to about 200 µL by ultrafiltration under centrifugation conditions (25°C, 1000 g, about 10 min). Finally, the volume was increased by diluting with PBS to a nucleic acid concentration of 10 µg/mL.

### [Experimental Example 1] Evaluation of particle properties of various mRNA-encapsulating LNPs

The particle size and particle surface potential of the LNPs of Examples 1 to 31 and Comparative Examples 1 to 4, which have different lipid compositions and were prepared in Production Examples 1 and 2, were measured by a dynamic scattering method (Zetasizer Nano; Malvern). The results are shown in Table 3 to Table 7. All LNPs had preferred particle size and preferred surface charge.

**[Table 3]**

| evaluation of VE, TS, SS-EC addition method | | | | | |
|---|---|---|---|---|---|
| | VE, TS, SS-EC addition method | lipid composition | particle size (nm) | PdI | surface potential (mV) |
| Ex. 1 | pre-addition method | SS-OP/VE/DOPC/Chol/DMG-PEG2000 = 47.7/9.1/6.8/36.4/1.5 | 84 | 0.14 | -1 |
| Ex. 2 | post-addition method 1 | SS-OP/VE/DOPC/Chol/DMG-PEG2000 = 47.7/9.1/6.8/36.4/1.5 | 93 | 0.13 | -1 |
| Ex. 3 | post-addition method 2 | SS-OP/VE/DOPC/Chol/DMG-PEG2000 = 47.7/9.1/6.8/36.4/1.5 | 112 | 0.18 | -1 |
| Ex. 4 | post-addition method 3 | SS-OP/VE/DOPC/Chol/DMG-PEG2000 = 47.7/9.1/6.8/36.4/1.5 | 92 | 0.16 | -1 |
| Ex. 5 | post-addition method 3 | SS-OP/VE/DOPC/Chol/DMG-PEG2000 = 50/4.8/7.1/38.1/1.5 | 119 | 0.12 | -3 |
| Ex. 6 | post-addition method 3 | SS-OP/VE/DOPC/Chol/DMG-PEG2000 = 43.8/16.7/6.2/33.3/1.5 | 128 | 0.17 | -2 |
| Ex. 7 | post-addition method 3 | SS-OP/TS/DOPC/Chol/DMG-PEG2000 = 50/4.8/7.1/38.1/1.5 | 85 | 0.13 | -4 |
| Ex. 8 | post-addition method 3 | SS-OP/TS/DOPC/Chol/DMG-PEG2000 = 47.7/9.1/6.8/36.4/1.5 | 89 | 0.10 | -7 |
| Ex. 9 | post-addition method 3 | SS-OP/TS/DOPC/Chol/DMG-PEG2000 = 43.8/16.7/6.2/33.3/1.5 | 91 | 0.12 | -6 |
| Ex. 10 | pre-addition method | SS-OP/VE/DOPC/Chol/DMG-PEG2000 = 43.8/16.7/6.2/33.3/1.5 | 90 | 0.12 | -3 |
| Ex. 11 | pre-addition method | SS-OP/TS/DOPC/Chol/DMG-PEG2000 = 43.8/16.7/6.2/33.3/1.5 | 88 | 0.12 | -10 |
| Ex. 12 | pre-addition method | SS-OP/SS-EC/DOPC/Chol/DMG-PEG2000 = 47.7/9.1/6.8/36.4/1.5 | 94 | 0.02 | -4 |
| Ex. 13 | post-addition method 3 | SS-OP/SS-EC/DOPC/Chol/DMG-PEG2000 = 47.7/9.1/6.8/36.4/1.5 | 107 | 0.18 | -12 |
| Comp. Ex. 1 | - | SS-OP/DOPC/Chol/DMG-PEG2000 = 52.5/7.5/40/1.5 | 87 | 0.12 | -1 |

(in Table, composition ratio is in mol%, particle size is zeta average)

**[Table 4]**

| evaluation of OP/EC gene expression, CTL activity, antibody production | | | | |
|---|---|---|---|---|
| | lipid composition | particle size (nm) | PdI | surface potential (mV) |
| Ex. 14 | SS-OP/SS-EC/DOPC/Chol/DMG-PEG2000 = 47.5/5/7.5/40/1.5 Luc-mRNA-encapsulating | 98 | 0.12 | -4 |
| Ex. 15 | SS-OP/SS-EC/DOPC/Chol/DMG-PEG2000 = 42.5/10/7.5/40/1.5 Luc-mRNA-encapsulating | 84 | 0.17 | -5 |
| Ex. 16 | SS-OP/SS-EC/DOPC/Chol/DMG-PEG2000 = 32.5/20/7.5/40/1.5 Luc-mRNA-encapsulating | 86 | 0.18 | -3 |
| Ex. 17 | SS-OP/SS-EC/DOPC/Chol/DMG-PEG2000 = 22.5/30/7.5/40/1.5 Luc-mRNA-encapsulating | 82 | 0.16 | -3 |
| Ex. 18 | SS-OP/SS-EC/DOPC/Chol/DMG-PEG2000 = 47.5/5/7.5/40/1.5 OVA-mRNA-encapsulating | 98 | 0.12 | -4 |
| Ex. 19 | SS-OP/SS-EC/DOPC/Chol/DMG-PEG2000 = 42.5/10/7.5/40/1.5 OVA-mRNA-encapsulating | 84 | 0.17 | -5 |
| Ex. 20 | SS-OP/SS-EC/DOPC/Chol/DMG-PEG2000 = 32.5/20/7.5/40/1.5 OVA-mRNA-encapsulating | 86 | 0.18 | -3 |
| Ex. 21 | SS-OP/SS-EC/DOPC/Chol/DMG-PEG2000 = 22.5/30/7.5/40/1.5 OVA-mRNA-encapsulating | 82 | 0.16 | -3 |
| Ex. 22 | SS-OP/SS-EC/DOPE/Chol/DMG-PEG2000 = 30/30/30/10/3 OVA-mRNA-encapsulating | 81 | 0.17 | -1 |
| Comp. Ex. 2 | SS-OP/DOPC/Chol/DMG-PEG2000 = 52.5/7.5/40/1.5 Luc-mRNA-encapsulating | 101 | 0.13 | -3 |
| Comp. Ex. 3 | SS-OP/DOPE/Chol/DMG-PEG2000 = 60/30/10/3 OVA-mRNA-encapsulating | 80 | 0.18 | 0 |

In all cases, SS-EC was added using the pre-addition method to prepare LNPs.
(in Table, composition ratio is in mol%)

**[Table 5]**

| Evaluation of influence of L/R ratio | | | | |
|---|---|---|---|---|
| | lipid composition | particle size (nm) | PdI | surface potential (mV) |
| Ex. 20 | SS-OP/SS-EC/DOPC/Chol/DMG-PEG2000 = 32.5/20/7.5/40/1.5 L/R=200 | 93 | 0.13 | -3 |
| Ex. 23 | SS-OP/SS-EC/DOPC/Chol/DMG-PEG2000 = 32.5/20/7.5/40/1.5 L/R=167 | 92 | 0.09 | -3 |
| Ex. 24 | SS-OP/SS-EC/DOPC/Chol/DMG-PEG2000 = 32.5/20/7.5/40/1.5 L/R=133 | 98 | 0.17 | -3 |
| Ex. 25 | SS-OP/SS-EC/DOPC/Chol/DMG-PEG2000 = 32.5/20/7.5/40/1.5 L/R=100 | 101 | 0.17 | -5 |
| Ex. 26 | SS-OP/SS-EC/DOPC/Chol/DMG-PEG2000 = 32.5/20/7.5/40/1.5 L/R=67 | 105 | 0.11 | -5 |
| Ex. 27 | SS-OP/SS-EC/DOPC/Chol/DMG-PEG2000 = 32.5/20/7.5/40/1.5 L/R=33 | 119 | 0.16 | -4 |

In all cases, SS-EC was added using the pre-addition method to prepare LNPs, and OVA-mRNA was encapsulated as nucleic acid.
(in Table, composition ratio is in mol%, L/R ratio is weight of mRNA for total lipid mol amount excluding DMG-PEG2000; unit is nmol/µg)

**[Table 6]**

| evaluation of IL-6 production | | | | |
|---|---|---|---|---|
| | lipid composition | particle size (nm) | PdI | surface potential (mV) |
| Ex. 28 | SS-OP/SS-EC/DOPC/Chol/DMG-PEG2000 = 47.5/5/7.5/40/1.5 not encapsulating nucleic acid | 89 | 0.07 | -1 |
| Ex. 29 | SS-OP/SS-EC/DOPC/Chol/DMG-PEG2000 = 42.5/10/7.5/40/1.5 not encapsulating nucleic acid | 88 | 0.06 | -2 |
| Ex. 30 | SS-OP/SS-EC/DOPC/Chol/DMG-PEG2000 = 32.5/20/7.5/40/1.5 not encapsulating nucleic acid | 89 | 0.03 | -2 |
| Ex. 31 | SS-OP/SS-EC/DOPC/Chol/DMG-PEG2000 = 22.5/30/7.5/40/1.5 not encapsulating nucleic acid | 83 | 0.06 | -2 |
| Comp. Ex. 4 | SS-OP/DOPC/Chol/DMG-PEG2000 = 52.5/7.5/40/1.5 not encapsulating nucleic acid | 92 | 0.07 | -1 |

In all cases, SS-EC was added using the pre-addition method to prepare LNPs.
(in Table, composition ratio is in mol%)

**[Table 7]**

| evaluation of influence of composition | | | | |
|---|---|---|---|---|
| | lipid composition | particle size (nm) | PdI | surface potential (mV) |
| Ex. 32 | SS-OP/SS-EC/DOPC/Chol/DMG-PEG2000 = 29.2/20/7.6/43.2/1.5 | 91 | 0.14 | -3 |
| Ex. 33 | SS-OP/SS-EC/DOPC/Chol/DMG-PEG2000 = 27.5/20/7.5/45/1.5 | 89 | 0.13 | -3 |

In all cases, SS-EC was added using the pre-addition method to prepare LNPs.
(in Table, composition ratio is in mol%)

### [Experimental Example 2] Evaluation of CTL activity using LNP containing VE (consideration of addition method)

### • subcutaneous administration to mice

OVA-mRNA-encapsulating LNP prepared according to the method described in Production Example 1 or Production Example 2 was diluted with PBS to an mRNA concentration of 2.0 µg/mL. The diluted mRNA-encapsulating LNP was subcutaneously administered to 6-week-old female C57/BL6J mice at a dose of 50 µL per mouse (mRNA dose: 0.1 µg per mouse).

### evaluation of CTL activity

One week after administration, the following CTL assay was performed to evaluate OVA-specific cytotoxic T lymphocyte (CTL) activity. The following composition was used as the culture medium for splenocytes: RPMI1640 500 mL, FBS 50 mL, 100 mM sodium pyruvate 5 mL, 1 M HEPES 5 mL, 55 mM 2-mercaptoethanol 500 µL, penicillin/streptomycin 5 mL (hereinafter medium).

Untreated mice were euthanized by cervical dislocation, and the spleens were harvested. The spleens were cut in the center, and spleen cells were removed using tweezers from the cut surface and suspended in a medium. The cell suspension medium was collected in a 50 mL tube through a 40 µm cell strainer and centrifuged (4°C, 500 g, 5 min). The supernatant was discarded, and 1 mL of Red Blood Cell Lysis Buffer (SIGMA) was added per mouse and left standing for 5 min. The cells were diluted 5-fold with a medium and centrifuged (4°C, 500 g, 5 min). The cells were further suspended in 10 mL of a medium and centrifuged (4°C, 500 g, 5 min). The cells were suspended in 30 mL of medium and the number of cells was counted. Thereafter, the mixture was passed through a 40 µm cell strainer, divided into equal amounts in two 50 mL tubes, centrifuged (4°C, 500 g, 5 min), and suspended in a medium at 1.0×10⁷ cells/mL. (1) One of the cell suspensions was added with 2 mM OVA epitope (consisting of the amino acid sequence of OVA positions 257-264) solution in an amount of 1/400 of the cell suspension, and left standing in a culture incubator for 1 hr. The epitope-pulsed group was centrifuged (4°C, 500 g, 5 min). The cells were washed with 10 mL of a medium and then with 10 mL of PBS, and the cells were counted again, suspended at 3.0×10⁷ cells/mL, and stained with CFSE at a final concentration of 5 µM to obtain "target cells". (2) The other cell suspension was incubated without adding the epitope and washed with a medium and PBS. The cells were suspended at 3.0×10⁷ cells/mL and stained with CFSE at a final concentration of 0.5 µM to obtain "control cells". The target cells and control cells were washed twice with a medium and twice with PBS, and suspended at 5.0×10⁷ cells/mL. 100 µL of the "target cells" and 100 µL of the "control cells" were mixed and administered to immunized mice. The two cells can be distinguished from each other based on the intensity of CFSE fluorescence. Twenty hours after administration of the cell mixture, spleens were harvested from the mice and subjected to flow cytometry analysis. The amount of the "target cells" present was corrected using the "control cells" to quantify epitope-specific CTL activity.

The evaluation results are shown in Fig. 1. Regardless of which LNP was used that was prepared by adding VE by which addition method, higher CTL activity was observed compared to Comparative Example 1. In particular, the LNPs prepared in Example 3 (post-addition method 2) or Example 4 (post-addition method 3) exhibited about 4-fold higher CTL activity compared to Comparative Example 1. CTL activity is an index for evaluating whether acquired immunity can be obtained, and higher CTL activity means that the adaptive immunity can be obtained more. Therefore, it was shown that the LNPs of Example 1 to Example 4 can enhance acquired immunity more than Comparative Example 1 of the conventional technique.

### [Experimental Example 3] Evaluation of CTL activity using LNP containing VE (consideration of amount of VE to be added)

### (1) subcutaneous administration to mice

According to the method described in [Experimental Example 2], OVA-mRNA-encapsulating LNP was subcutaneously administered to mice (mRNA dose was 0.1 µg per mouse).

### (2) evaluation of CTL activity

According to the method described in [Experimental Example 2], CTL activity was evaluated. The results thereof are shown in Fig. 2. Regardless of which LNP of Example 4 to Example 6 was used, higher CTL activity was observed compared to Comparative Example 1. In particular, the LNP of Example 6 containing 16.7 mol% VE exhibited about 2.3-fold higher CTL activity compared to the LNP of Comparative Example 1. The results show that the LNPs of Examples 4 to 6 can enhance acquired immunity more than Comparative Example 1 of the conventional technique.

### [Experimental Example 4] Evaluation of CTL activity using LNP containing TS (consideration of amount of TS to be added)

### • subcutaneous administration to mice

According to the method described in [Experimental Example 2] except for reducing the mRNA concentration of the LNP to be prepared by half, OVA-mRNA-encapsulating LNP was subcutaneously administered to mice (mRNA dose was 0.05 µg per mouse).

### · evaluation of CTL activity

According to the method described in [Experimental Example 2], CTL activity was evaluated. The results thereof are shown in Fig. 3. Regardless of which LNP of Example 7 to Example 9 was used, higher CTL activity was observed compared to Comparative Example 1. In particular, the LNP of Example 9 containing 16.7 mol% TS exhibited about 5.4-fold higher CTL activity compared to the LNP of Comparative Example 1. The results show that the LNPs of Examples 7 to 9 can enhance acquired immunity more than Comparative Example 1 of the conventional technique.

### [Experimental Example 5] Evaluation of CTL activity using various LNPs (comparison of pre-addition method and post-addition method)

### • subcutaneous administration to mice

### [Production Example]

According to the method described in [Experimental Example 2] except for reducing the mRNA concentration of the LNP to be prepared to one-fourth, OVA-mRNA-encapsulating LNP was subcutaneously administered to mice (mRNA dose was 0.025 µg per mouse).

### · evaluation of CTL activity

According to the method described in [Experimental Example 2], CTL activity was evaluated. The results thereof are shown in Fig. 4. Higher CTL activity was observed compared to Comparative Example 1 even when the LNPs of Example 6 and Example 9 to Example 13 were used, and it was clarified that the CTL activity can be enhanced regardless of the addition method. The results show that any addition method can enhance acquired immunity more than Comparative Example 1 of the conventional technique. In particular, it was clarified that the LNPs of Example 12 and Example 13 each containing 9.1 mol% SS-EC can enhance the CTL activity more than the conditions in which 16.7 mol% of VE or TS was added.

### [Experimental Example 6] Evaluation of gene expression activity using LNP containing SS-EC (consideration of amount of SS-EC to be added)

### • subcutaneous administration to mice

Luc-mRNA-encapsulating LNP prepared according to the method described in Production Example 2 was diluted with PBS to an mRNA concentration of 5.0 µg/mL. The diluted mRNA-encapsulating LNP was subcutaneously administered to 6-week-old female C57/BL6J mice at a dose of 200 µL per mouse (mRNA dose: 1.0 µg per mouse).

### • measurement of gene expression activity

5.5 hours after administration of the LNPs, a Luciferin solution was intraperitoneally administered to the mice. 30 min after administration, gene expression activity at the administration site was evaluated using IVIS. The results are shown in Fig. 5. It was clarified that all LNPs of Example 14 to Example 17 show equivalent gene expression activity compared to the LNP of Comparative Example 2. These results demonstrate that the LNPs of Example 14 to Example 17 have nucleic acid delivery efficiency equivalent to that of Comparative Example 2.

### [Experimental Example 7] Evaluation of CLT activity using LNP containing SS-EC (consideration of amount of SS-EC to be added)

### • subcutaneous administration to mice

According to the method described in [Experimental Example 2] except for reducing the mRNA concentration of the LNP to be prepared by half, OVA-mRNA-encapsulating LNP was subcutaneously administered to mice (mRNA dose was 0.05 µg per mouse).

### · evaluation of CTL activity

According to the method described in [Experimental Example 2], CTL activity was evaluated. The results thereof are shown in Fig. 6. Regardless of which LNP of Example 18 to Example 21 was used, higher CTL activity was observed compared to Comparative Example 1. The results show that any addition amount can enhance acquired immunity more than Comparative Example 1 of the conventional technique. In particular, the LNP of Comparative Example 21 containing 30 mol% SS-EC exhibited 4.3-fold higher CTL activity compared to Comparative Example 1. Although the nucleic acid delivery efficiency in [Experimental Example 6] was equivalent to that of the LNP of the Comparative Example, the LNPs of Example 18 to Example 21 showed enhanced CTL activity. Thus, it was shown that these LNPs can efficiently enhance acquired immunity.

### [Experimental Example 8] Evaluation of in vivo antibody production using LNP containing SS-EC (consideration of amount of SS-EC to be added)

### • intramuscular administration to mice

OVA-mRNA-encapsulating LNP prepared according to the method described in Production Example 2 was diluted with PBS to an mRNA concentration of 30 µg/mL. The LNP was intramuscularly administered to 6-week-old female Balb/c mice at a dose of 50 µL per mouse (mRNA dose: 1.5 µg per mouse). Fourteen days after administration, LNP of the same composition was administered to the mice.

### • evaluation of in vivo antibody production

Fourteen days after the second administration, blood was collected from the mice and serum was recovered. The amount of anti-OVA antibody contained in the serum was quantified by ELISA.

The results thereof are shown in Fig. 7. Regardless of which LNP of Example 19 to Example 21 was used, an antibody production amount higher than that in Comparative Example 1 was observed. The production of antibody against an antigen (OVA) is an index showing whether acquired immunity can be obtained, and a higher amount of antibody production indicates a more efficient enhancement of acquired immunity. Therefore, it was shown that the LNPs of Example 19 to Example 21 can enhance acquired immunity more than Comparative Example 1 of the conventional technique.

### [Experimental Example 9] Evaluation of in vivo antibody production using LNP containing SS-EC - 2

### • intramuscular administration to mice

According to the method described in [Experimental Example 8], OVA-mRNA-encapsulating LNP was intramuscularly administered to mice (mRNA dose was 1.5 µg per mouse).

### • evaluation of in vivo antibody production

According to the method described in [Experimental Example 8], the amount of anti-OVA antibody contained in the serum was quantified. The results thereof are shown in Fig. 8. It was shown that the LNP of Example 22 produced an antibody, while no antibody was produced in Comparative Example 3.

### [Experimental Example 10] Evaluation of in vivo antibody production using LNP containing SS-EC - 3 (investigation of effect of ratio of nucleic acid amount relative to total lipid)

### • intramuscular administration to mice

According to the method described in [Experimental Example 8], OVA-mRNA-encapsulating LNP having a different ratio of nucleic acid amount relative to total lipid was prepared and intramuscularly administered to mice (mRNA dose was 1.5 µg per mouse) .

### • evaluation of in vivo antibody production

According to the method described in [Experimental Example 8], the amount of anti-OVA antibody contained in the serum was quantified. The results thereof are shown in Fig. 9. It was shown that the antibody production amount was high regardless of which LNP of Example having a different ratio of nucleic acid amount relative to the total lipid (Example 20 and Example 23 to Example 27) was used.

### [Experimental Example 11] Evaluation of cytokine production using LNP containing SS-EC

### • subcutaneous administration to mice

According to the method described in [Production Example 2], empty LNPs not encapsulating nucleic acid were prepared and subcutaneously administered to mice (lipid amount 200 nmol).

### • evaluation of cytokine production

Six hours after LNP administration, blood was collected and serum was recovered. The amount of IL-6 contained in the serum was quantified by ELISA. The results thereof are shown in Fig. 10. It was shown that the LNPs of Example 28 to Example 31 have higher concentrations of IL-6 in the serum than Comparative Example 4. IL-6 is a cytokine produced by cells such as T cells and macrophages, and plays a role of enhancing acquired immunity. In other words, high concentrations of IL-6 in serum indicate that acquired immunity is efficiently enhanced.

### [Industrial Applicability]

The lipid nanoparticles of the present invention are useful as a constituent element of nucleic acid vaccines to afford acquired immunity.

This application is based on a patent application No. 2022-114395 filed in Japan, the contents of which are encompassed in full herein.

## Claims

1. A lipid nanoparticle comprising
(A) an ionic lipid represented by the formula (1): 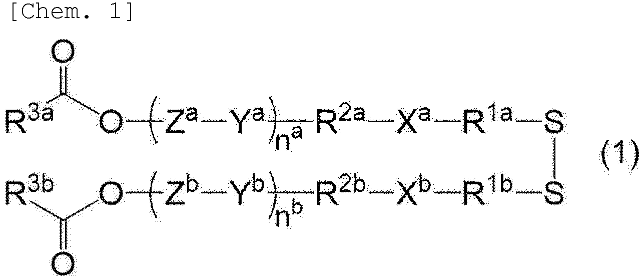 (in the formula (1),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1, and
R^{3a} and R^{3b} are each independently
(a) a group such that R^{3a}-COO- and R^{3b}-COO- are each a residue derived from a reaction product of a sterol derivative having a hydroxyl group, and succinic anhydride or glutaric anhydride, or
(b) an aliphatic hydrocarbon group having 1 to 40 carbon atoms, an alkyl group having 3 to 40 carbon atoms and a cyclopropane ring, or
(c) a group represented by the formula (2):
R⁹-O-CO-(CH₂)ₐ- (2)
(in the formula (2),
R⁹ is an aliphatic hydrocarbon group having 2 to 20 carbon atoms, and
a is an integer of 2 to 10)),
(B) vitamin E and/or a derivative thereof,
(C) phospholipid,
(D) cholesterol, and
(E) PEG lipid.

2. The lipid nanoparticle according to claim 1, wherein the vitamin E is selected from the group consisting of α-tocopherol, β-tocopherol, α-tocotrienol, and β-tocotrienol.

3. The lipid nanoparticle according to claim 1, wherein the vitamin E derivative is selected from the group consisting of α-tocopherol succinate, α-tocopherol glutarate, β-tocopherol succinate, β-tocopherol glutarate, α-tocotrienol succinate, α-tocotrienol glutarate, β-tocotrienol succinate, and β-tocotrienol glutarate.

4. The lipid nanoparticle according to claim 1, wherein the vitamin E derivative is a compound represented by the formula (3): 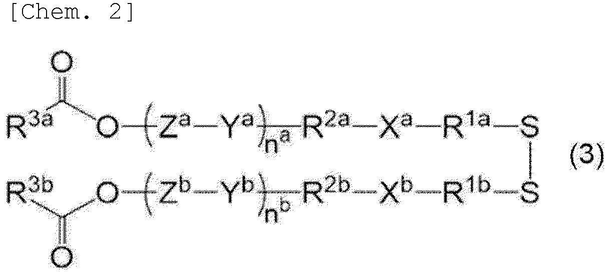 (in the formula (3),
R^{1a} and R^{1b} are each independently an alkylene group having 1 to 6 carbon atoms,
X^{a} and X^{b} are each independently an acyclic alkyl tertiary amino group having 1 to 6 carbon atoms and one tertiary amino group, or a cyclic alkylene tertiary amino group having 2 to 5 carbon atoms and 1 to 2 tertiary amino groups,
R^{2a} and R^{2b} are each independently an alkylene group or an oxydialkylene group each having not more than 8 carbon atoms,
Y^{a} and Y^{b} are each independently an ester bond, an amide bond, a carbamate bond, an ether bond or a urea bond,
Z^{a} and Z^{b} are each independently a divalent group derived from an aromatic compound having 3 to 16 carbon atoms and at least one aromatic ring, and optionally having a hetero atom,
n^{a} and n^{b} are each independently 0 or 1,
R^{3a} and R^{3b} are each independently a group such that R^{3a}-COO- and R^{3b}-COO- are each a residue derived from a reaction product of a hydroxyl group of vitamin E, and succinic anhydride or glutaric anhydride).

5. The lipid nanoparticle according to claim 1, wherein the vitamin E derivative is a compound represented by the formula (4): 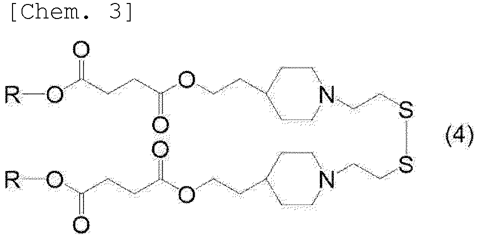 (in the formula (4),
R is a group such that RCOO(CH₂)₂COO- is a residue of a reaction of a hydroxyl group of α-tocopherol, β-tocopherol, α-tocotrienol, or β-tocotrienol and succinic anhydride) or
the formula (5): 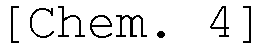 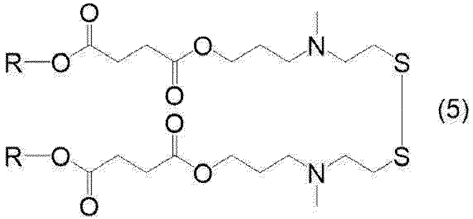 (in the formula (5),
R is a group such that RCOO(CH₂)₂COO- is a residue of a reaction of a hydroxyl group of α-tocopherol, β-tocopherol, α-tocotrienol, or β-tocotrienol and succinic anhydride).

6. The lipid nanoparticle according to any one of claims 1 to 5, wherein the ionic lipid is a compound represented by the following formula:

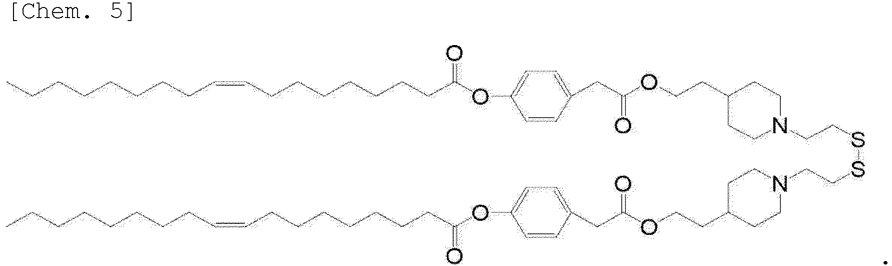

7. The lipid nanoparticle according to claim 5, comprising 20 to 55 mol% of the ionic lipid, 4.5 to 35 mol% of the vitamin E and/or a derivative thereof, 5 to 35 mol% of the phospholipid, 10 to 50 mol% of the cholesterol, and 0.5 to 4 mol% of the PEG lipid, with respect to the total of the ionic lipid, the vitamin E and/or a derivative thereof, the phospholipid, and the cholesterol.

8. The lipid nanoparticle according to claim 6, comprising 20 to 55 mol% of the ionic lipid, 4.5 to 35 mol% of the vitamin E and/or a derivative thereof, 5 to 35 mol% of the phospholipid, 10 to 50 mol% of the cholesterol, and 0.5 to 4 mol% of the PEG lipid, with respect to the total of the ionic lipid, the vitamin E and/or a derivative thereof, the phospholipid, and the cholesterol.

9. The lipid nanoparticle according to claim 8, wherein the phospholipid is 1,2-diacyl-sn-glycero-3-phosphocholine or 1,2-diacyl-sn-glycero-phosphoethanolamine.

10. The lipid nanoparticle according to claim 8, wherein the phospholipid is selected from the group consisting of 1,2-dioleoyl-sn-glycero-3-phosphocholine (DOPC), 1,2-distearoyl-snglycero-3-phosphocholine (DSPC), and 1,2-dioleoyl-sn-glycero-3-phosphoethanolamine (DOPE).

11. The lipid nanoparticle according to claim 8, wherein the PEG lipid is a dimyristoylglycerol PEG represented by the formula (6):
CH₂(OR⁶)-CH(OR⁷)-CH₂(OR⁸) (6)
(in the formula (6),
any two of R⁶, R⁷ and R⁸ are myristoyl groups, and the remaining one is an alkyl group having 1 to 6 carbon atoms which is linked via a polyethylene glycol chain having a number average molecular weight of 1,000 to 3,000).

12. The lipid nanoparticle according to claim 10, wherein the PEG lipid is a dimyristoylglycerol PEG represented by the formula (6):
CH₂(OR⁶)-CH(OR⁷)-CH₂(OR⁸) (6)
(in the formula (6),
any two of R⁶, R⁷ and R⁸ are myristoyl groups, and the remaining one is an alkyl group having 1 to 6 carbon atoms which is linked via a polyethylene glycol chain having a number average molecular weight of 1,000 to 3,000).

13. A nucleic acid-encapsulating lipid nanoparticle comprising the lipid nanoparticle according to claim 1 and a nucleic acid encapsulated therein.

14. A method for production the nucleic acid-encapsulating lipid nanoparticle according to claim 13, comprising a vitamin addition step for adding the vitamin E and/or a derivative thereof to the ionic lipid, the phospholipid, the cholesterol, and the PEG lipid.

15. The production method according to claim 14, wherein the vitamin addition step further comprises, after addition of the vitamin E and/or a derivative thereof to an alcohol solution comprising the ionic lipid, the phospholipid, the cholesterol, and the PEG lipid, a step of mixing with a buffer comprising the nucleic acid.

16. The production method according to claim 14, wherein the vitamin addition step comprises, after mixing an alcohol solution comprising the ionic lipid, the phospholipid, the cholesterol, and the PEG lipid with a buffer comprising the nucleic acid, adding the vitamin E and/or a derivative thereof.

17. A method for imparting a living body with an acquired immunity to an antigen protein, comprising administering the lipid nanoparticle according to claim 1 encapsulating a nucleic acid that expresses the antigen to the living body.
